# FASCICULE DE BREVET EUROPEEN

(11) **EP 1 660 019 B1**
(45) Date de publication et mention de la délivrance du brevet: **14.07.2010**
(21) Numéro de dépôt: 03769573.1
(22) Date de dépôt: 05.09.2003
(51) Int. Cl.: A61K 6/093

(54) **COMPOSITION DENTAIRE POLYMERISABLE ET A HAUTE TENEUR EN CHARGE**
POLYMERISIERBARE DENTALZUSAMMENSETZUNG MIT HOHEM FÜLLSTOFFANTEIL
POLYMERIZABLE DENTAL COMPOSITION HAVING A HIGH CONTENT OF LOADING MATERIAL

(43) Date de publication de la demande: 31.05.2006
(73) Titulaire: Bluestar Silicones France, 69486 Lyon (FR)
(72) Inventeur: FRANCES, Jean-Marc, F-69330 Meyzieu (FR)
(74) Mandataire: Mekki, Boualem
(86) Numéro de dépôt international: PCT/FR2003/002649
(87) Numéro de publication internationale: WO 2005/027857

(56) Documents cités:
- EP-A- 0 403 197
- EP-A- 0 867 443
- EP-A2- 0 687 443
- EP-A2- 0 970 680
- WO-A-00/19967
- WO-A-92/16183
- DE-A- 19 754 442
- US-A- 4 795 796

## Description

Le domaine de l'invention est celui des compositions dentaires. Plus précisément, les compositions dentaires mises au point dans le cadre de la présente invention sont utilisables pour la réalisation de prothèses dentaires et pour la restauration dentaire.

Ces compositions dentaires sont classiquement des résines époxy, ou silicones photopolymérisables ou des résines acrylates polymérisables par voie radicalaire. Ces compositions incluent en outre des charges particulaires de renfort (e.g. en silice hydrophobisée), des photoamorceurs et éventuellement des photosensibilisateurs s'agissant des compositions cationiques et des initiateurs radicalaires pour ce qui concerne les compositions radicalaires, voire d'autres additifs fonctionnels tels que des pigments ou des stabilisants.
Une fois mélangées, ces compositions sont mises en forme puis photoréticulées en une masse de structure analogue à celle des dents.

Le fait que la charge soit constituée de particules très fines (≈ 0,05 µm) et de grande surface spécifique, est un facteur limitant de son taux d'incorporation dans la résine. Cette dernière a en effet une capacité d'absorption limitée. Il en résulte que les taux de charges de telles compositions atteignent rarement plus de 45 % en volume.
Cela pénalise donc la fonction de renfort mécanique assignée à la charge particulaire.
Cette dernière peut également interagir avec les fonctions réactives des espèces (photo)polymérisables/réticulables et être ainsi à l'origine de problèmes d'instabilité de la composition dentaire.

Le brevet US-B-6,306,926 concerne des compositions dentaires à base de résines époxy (e.g. polyTetraHydroFurane, UVR^{®} 6105, EPON^{®} 828, GY281^{®}), oxétane, ou vinyléther, entre autres, polyméri-sables/réticulables, par voie cationique et sous irradiation, et éventuellement des résines (méth)acrylate polymérisables par voie radicalaire. Outre les inducteurs de polymérisation que sont les photoamorceurs cationiques et éventuellement les initiateurs radicalaires selon le cas, ces compositions comprennent une charge minérale microparticulaire, radioopaque, qui est sélectionnée parmi les composés métalliques suivants : oxydes, halogénures, borates phosphates,silicates, carbonates, germanates, tétrafluoroborates, hexafluoro-phosphates, ayant un point isoélectrique inférieur à 7. Cette composition est telle que sa dureté Barcol d'au moins 10, après 30 min de polymérisation cationique à 25°C.

Ces résines ont l'inconvénient de ne pas être parfaitement transparentes au rayonnement actinique d'activation de la polymérisation actinique UV-visible, ce qui est dommageable à la cinétique réactionnelle et donc limite les possibilités d'obtenir des matériaux photoréticulés très épais.

La demande de brevet FR-A-2 784 025 vise à remédier à cela en proposant des compositions dentaires à base de résines silicones polyméri-sables/réticulables, par voie cationique et sous irradiation suivie ou non d'une post-réticulation thermique. Ces résines silicones sont à fonctionnalités oxirane (époxyde, oxétane...) ou vinyléther... De telles compositions comprennent un ou plusieurs polydiméthylsiloxanes réticulables et/ou polymérisables par voie cationique et porteurs à au moins l'une de leurs extrémités de fonctions réactives de formule : 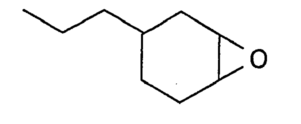 une quantité efficace d'au moins un amorceur de type borate d'onium: 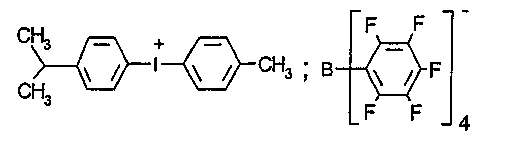 au moins un photosensibilisateur, et une charge dentaire ou de renforcement inerte à base de polyméthacrylate de méthyle ou de silice de combustion traitée hexaméthyldisilazane de surface spécifique 200 m2/g, présente dans la composition dans une proportion d'au moins 10% en poids par rapport au poids total de la composition. Ces compositions dentaires sont destinées à la fabrication de prothèses ou d'appareils dentaires.
Ces silicones présentent l'avantage par rapport à des résines organiques réticulant par voie cationique d'être très transparents à la lumière UV-visible et donc de permettre l'obtention de matériaux très épais (plusieurs millimètres d'épaisseur) photoréticulés en très peu de temps (inférieur à une minute) avec une lampe UV émettant dans le domaine du visible >400 nm.
Cependant ces silicones sont formulés avec des charges de renforcement à caractère acide de Lewis ou de Bronsted, telles que des quartz ou des silices de combustion de très faible granulométrie, dont les silanols de surface et/ou l'eau résiduelle, réagissent avec les fonctions cationiques. De telles formulations silicones sont donc instables au stockage des compositions.

Outre ce problème d'instabilité engendré par les charges, ces compositions dentaires silicones restent perfectibles en ce qui concerne l'accroissement du taux de charge, de manière à permettre l'amélioration des propriétés mécaniques.

On connaît par ailleurs, au travers de la demande de brevet EP-A-1 050 291 des compositions dentaires fortement chargées, présentées comme étant dotées de bonnes propriétés mécaniques et comprenant de 10 à 70 % en volume de charge (e.g. silice de combustion) de granulométrie Φm comprise 0,05-0,5 µm (moins de 50 % en volume de particules de diamètre Φ > 0,50 µm, un monomère acrylique photopolymérisable par voie radicalaire et un dispersant de type ester d'acide phosphorique de formule :

R-[-CO-(CH₂)₅-O-]ₙ-PO₃H₂

Un tel enseignement relatif à des compositions dentaires radicalaires n'est nullement transposable aux compositions dentaires cationiques à base de silicone. En effet, les dispersants R-[-CO-(CH₂)₅-O-]ₙ-PO₃H₂ ne sont pas adaptés aux compositions cationiques, notamment car ils contiennent un résidu acide RPO₃H₂ important qui réagit en présence de fonctions oxiranes et nuit à la stabilité de la composition.

Il apparaît donc que l'art antérieur n'apporte pas de solution satisfaisante au double problème de stabilisation des compositions dentaires à base de motifs polymérisables par voie cationique sous UV (par exemple des oxiranes) et de dispersion de quantités importantes de charges dans la résine.

L'un des objectifs essentiels de la présente invention est donc de remédier à cela en fournissant de nouvelles compositions dentaires à base de motifs polymérisables par voie cationique sous UV (par exemple des oxiranes), ne présentant pas les inconvénients de l'art antérieur sur le plan de la stabilité et du taux de charge limité.

Un autre objectif essentiel de la présente invention est de fournir de nouvelles compositions dentaires cationiques, polymérisables et/ou réticulables en environnement oral, qui soient non seulement stables et fortement chargées (e.g. ≥50 %) mais qui aient également des qualités nettement améliorées, notamment en ce qui concerne la réduction très nette du phénomène de retrait des compositions dentaires utilisées pour la réalisation de prothèses dentaires ou de matériaux de restauration dentaire.

Un autre objectif essentiel de la présente invention est de fournir de nouvelles compositions dentaires cationiques, polymérisables et/ou réticulables en environnement oral, qui soient non seulement stables et fortement chargées (e.g. ≥50 %) mais qui aient également l'avantage d'être très transparentes à la lumière UV-visible et donc de permettre l'obtention de matériaux très épais (plusieurs millimètres d'épaisseur) photoréticulés en très peu de temps (inférieur à une minute) avec une lampe UV émettant dans le domaine du visible > 400 nm.

Un autre objectif essentiel de la présente invention est de fournir de nouvelles compositions dentaires cationiques, polymérisables et/ou réticulables en environnement oral, qui soient non seulement stables et fortement chargées (e.g. ≥50 %) mais qui soient également faciles à préparer et économiques.

Ces objectifs, parmi d'autres, sont atteints par la présente invention qui concerne tout d'abord une composition dentaire comprenant :
**(1)** au moins un composé réactif par voie cationique et sous activation, de préférence actinique ;
**(2)** au moins une charge dentaire présente dans une proportion d'au moins 10% en poids par rapport au poids total de la composition ;
**(3)** au moins un dispersant à base d'au moins un polymère ou copolymère organique choisis parmi ceux dont l'indice d'amine est inférieur ou égal à 100 mg de potasse par gramme de dispersant et sélectionné dans le groupe comprenant les copolymères polyuréthan/acrylate ; éventuellement salifiés par un alkylammonium ;
**(4)** au moins un photoamorceur cationique ;
**(5)** et éventuellement au moins un photosensibilisateur.

Il est du mérite des inventeurs d'avoir montré, de manière surprenante et inattendue, qu'il est possible d'utiliser pendant la formulation des dispersants (co)polymères judicieusement sélectionnés, qui permettent de traiter la surface de la charge et d'augmenter ainsi le taux de charge et donc in fine de renforcer le matériau sans nuire à la stabilité du composite.
Cette stabilité se traduit par une durée limite d'utilisation de plusieurs mois ou années.
Ces dispersants permettent de fluidifier le matériau sans l'opacifier tout en gardant une très grande stabilité des compositions. On peut de ce fait de l'invention utiliser des taux de charge traitée de très faible granulométrie très importants ≥ 50 %. Cette solution technique est d'autant plus intéressante qu'elle est économiquement viable et facile à mettre en oeuvre.
Il est également intéressant de noter que cette composition donne satisfaction en termes de limitation du retrait après polymérisation/réticulation, ce qui est tout à fait appréciable dans l'application dentaire.

En général, l'activation photochimique est réalisée sous rayonnement U.V. Plus particulièrement, on utilise un rayonnement U.V. de longueur d'onde de l'ordre de 200 à 500 nm pour la réalisation de prothèses dentaires et un rayonnement U.V. visible de longueur d'onde supérieur à 400 nm pour la réalisation de matériaux de restauration. Une longueur d'onde supérieure à 400 nm permet la réticulation et/ou polymérisation en environnement oral.

L'activation par voie actinique (photochimique) peut être avantageu-sement complétée (voire remplacée) par une activation thermique.

De préférence, l'indice d'amine du dispersant (3) est inférieur ou égal à 60, et plus préférablement encore compris entre 0,1 et 50 mg de potasse par gramme de dispersant (3).

Avantageusement, l'indice d'acide du dispersant est inférieur ou égal à 200, de préférence inférieur ou égal à 100, et plus préférablement compris entre 1 et 60 mg de potasse par gramme de dispersant.
Suivant une caractéristique avantageuse de l'invention, la charge dentaire (2) est présente à raison de 10 à 85 % en poids ou en volume.

Conformément à l'invention, le dispersant (3) est sélectionné dans le groupe comprenant : les copolymères polyuréthanne/acrylate éventuellement salifiés par un alkylammonium, les copolymères acryliques éventuellement salifiés, par un alkylammonium, les monodiesters d'acides carboxyliques, les polyesters, les polyéthers, les polyuréthannes, les polyuréthannes modifiés, les polyol-polyacrylates, leurs copolymères ou leurs mélanges.

Les dispersants commercialisés sous la marque DISPERBYK^{®} (de la société Byk) ou SOLSPERSE^{®} (de la société Avecia) conviennent particulièrement à l'invention.
On peut citer en particulier et à titre d'exemples, les produits commerciaux : Disperbyk^{®} 164, Disperbyk^{®} 161, Disperbyk^{®}166, Disperbyk^{®}2070, Disperbyk^{®} 9075, Disperbyk^{®} 9076.
Le brevet US-B-5,882,393 décrit des dispersants à base de polyuréthan-nes/imidazoles-acrylates ou époxydes.
Le brevet US-B-5,425,900 décrit des dispersants à base de polyuréthannes. & Le brevet US-B-4,795,796 décrit des dispersants à base de polyuréthannes/ polyoxyalkylène-glycolmonoalkyléther.
La demande de brevet WO-A-99/56864 décrit des dispersants à base de polyuréthannes/poly(oxyalkylène-carbonyle): dérivés de ε-caprolactone et de δ-valérolactone.
Le brevet EP-B-0 403 197 décrit des dispersants de type polyol-polyacrylate greffé comprenant un copolymère statistique polyuréthanne/polyvinyli-que/polyacrylate et un polyoxyalkylène polyéther.

Quantitativement parlant, le dispersant (3) est présent à raison de 50 ppm à 1 %, de préférence 100 ppm à 5000 ppm.

De préférence, le composé réactif par voie cationique (1) est choisi dans le groupe de monomères et/ou (co)polymères comprenant:
les époxy, les vinyles éthers, les oxétanes, les spiroorthocarbonates, les spiro-orthoesters et leurs associations.

Plus préférentiellement encore, le composé réactif par voie cationique (1) est constitué par au moins un oligomère ou polymère silicone réticulable et/ou polymérisable, liquide à température ambiante ou thermofusible à température inférieure à 100°C, et comprenant :
□ au moins un motif de formule (F*S*) *:* 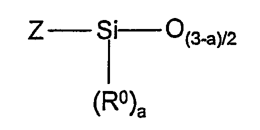 dans laquelle :
   - a = 0, 1 ou 2,
   - R⁰, identique ou différent, représente un radical alkyle, cycloalkyle, aryle, vinyle, hydrogéno, alcoxy, de préférence un alkyle inférieur en C₁-C₆,
   - Z, identique ou différent, est un substituant organique comportant au moins une fonction réactive époxy, et/ou alcénylether et/ou oxétane et/ou dioxolane et/ou carbonate,
□ et au moins deux atomes de silicium.

Ce polymère ou oligomère silicone (1) présente l'avantage par rapport à des résines organiques réticulant par voie cationique d'être transparente à la lumière UV-visible et donc son utilisation permet d'obtenir des matériaux très épais et dont la photoréticulation s'effectue en peu de temps.

Les fonctions réactives Z du polymère ou oligomère silicone (1) peuvent être très variées. Toutefois, des compositions dentaires particulièrement intéressantes sont obtenues lorsque l'oligomère ou polymère silicone (1) comprend au moins un motif (FS) dans lequel Z représente un substituant organique Z1 comportant au moins une fonction réactive époxy, et/ou dioxolane, et de préférence au moins une fonction réactive époxy.

Selon deux alternatives avantageuses de la présente invention, l'oligomère ou polymère silicone (1) avec au moins une fonction réactive Z1 époxy, et/ou dioxolane, et de préférence au moins une fonction réactive époxy peut:
(i) soit comporter uniquement ce(s) type(s) de fonction(s) réactive(s) Z1 ;
(ii) ou soit comporter d'autres fonctions réactives Z telles que les fonctions réactives Z2 alcénylether, oxétane et/ou carbonate.

Dans le cas de la première alternative (i), la composition dentaire peut également comprendre d'autres oligomères et/ou polymères silicones comportant d'autres fonctions réactives Z2 telles que les fonctions alcénylether, oxétane et/ou carbonate et éventuellement des fonctions réactives Z1.

A titre d'exemples de fonctions réactives Z, celles ci peuvent être notamment choisies parmi les radicaux suivants : 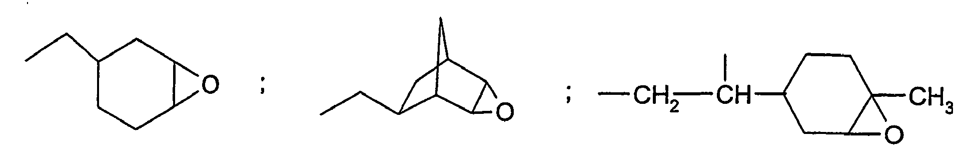 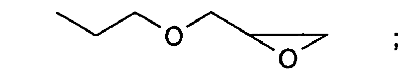 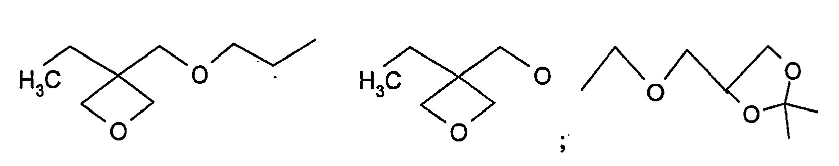 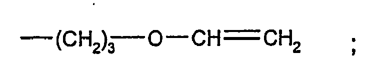 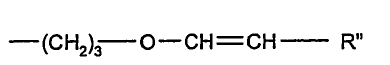 - avec R" représentant un radical alkyle linéaire ou ramifié en C₁-C₆.

Selon une deuxième variante avantageuse de la présente invention, le polymère ou oligomère silicone est constitué par au moins une silicone de formule moyenne suivante :

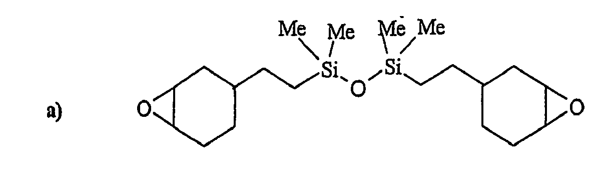

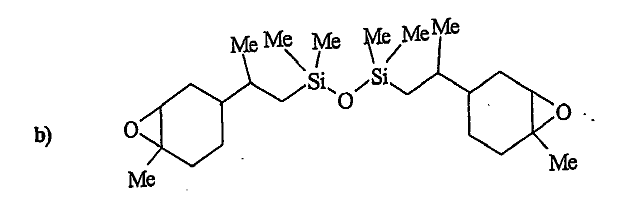

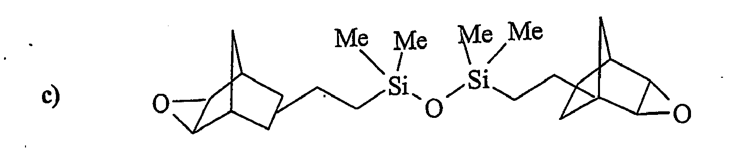

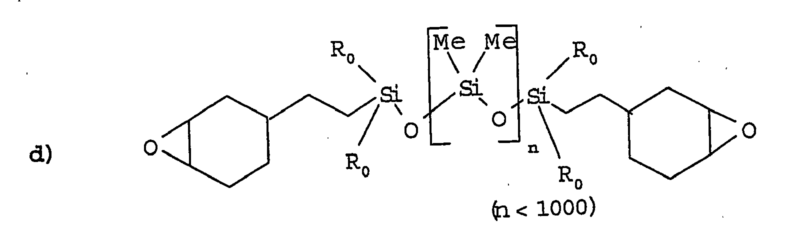

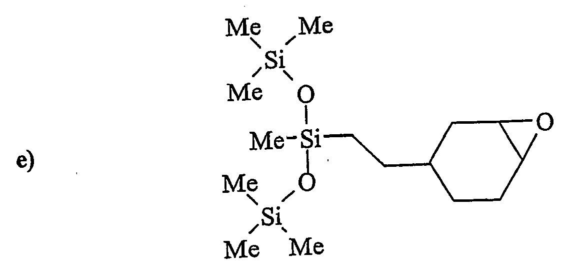

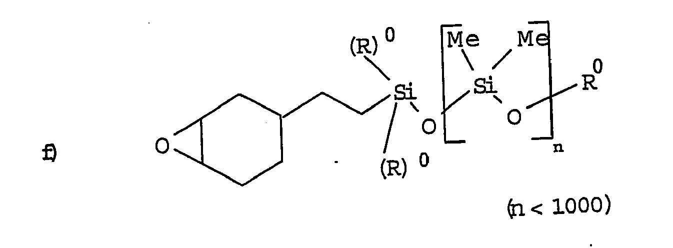

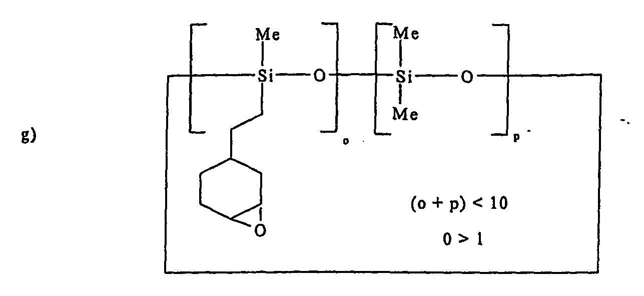

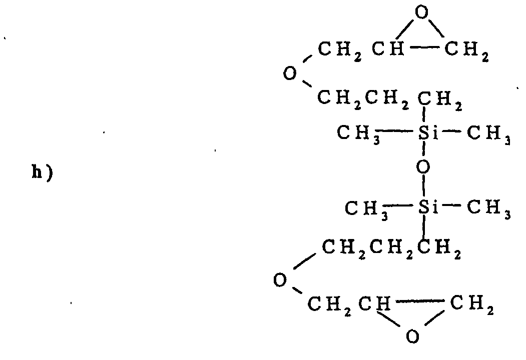

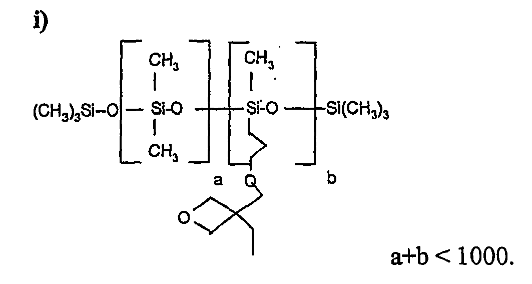

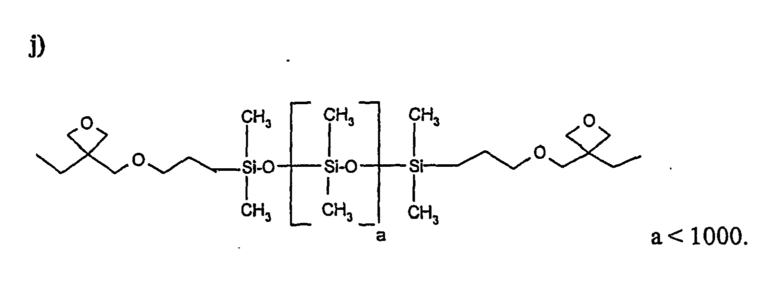

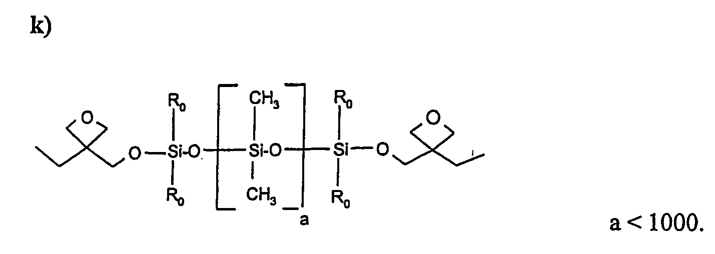

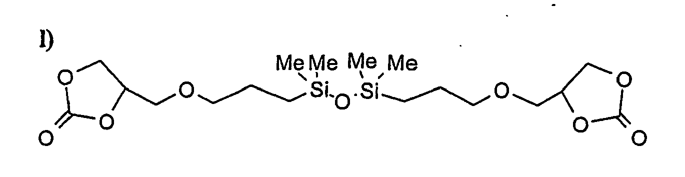

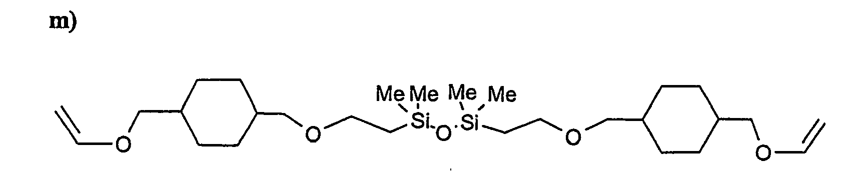

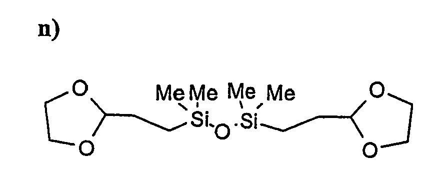

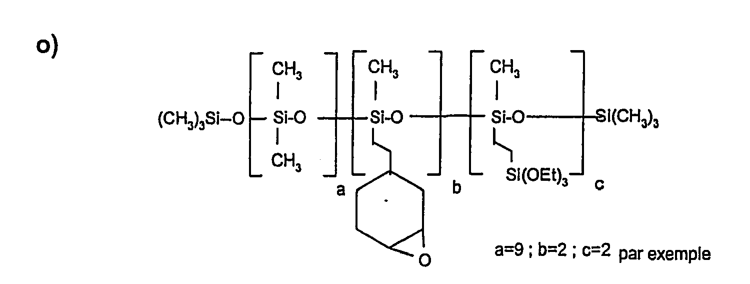

Différents types de charges (2) sont utilisables pour préparer les compositions selon l'invention. Les charges sont choisies en fonction de l'utilisation finale de la composition dentaire : celles-ci affectent d'importantes propriétés telles que l'apparence, la pénétration du rayonnement U.V., ainsi que les propriétés mécaniques et physiques du matériau obtenu après réticulation et/ou polymérisation de la composition dentaire.

Comme charge de renforcement, on peut utiliser des charges de silice de pyrogénation traitée ou non, des charges de silice amorphe, du quartz, des verres ou des charges non vitreuses à base d'oxydes de zirconium, de baryum, de calcium, de fluor, d'aluminium, de titane, de zinc, des borosilicates, des aluminosilicates, du talc, des sphérosil, du trifluorure d'yterbium, des charges à base de polymères sous forme de poudre broyée tel que des polyméthacrylates de méthyle inertes ou fonctionnalisés, des polyépoxydes ou des polycarbonates.

A titre d'exemple, on citera:
- des charges inertes à base de polyméthacrylate de méthyle LUXASELF de la société UGL utilisables dans le domaine dentaire et pigmentées en rose,
- des charges de silice de combustion traitée hexaméthyldisilazane de surface spécifique 200 m²/g,
- des charges de silice de combustion non traitée (« aerosil » AE200 commercialisée par DEGUSSA),
- des quartz ou verres d'oxydes de silicium.

Selon une variante avantageuse de l'invention, les charges et en particulier les charges de silice, sont traitées avant utilisation à 120°C avec une quantité inférieure à 10% p/p de silicone comprenant au moins un motif de formule (XXIII) : 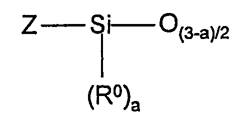
- tel que Z' a la même définition que Z
- a= 0,1 ,2 ou 3
- avec au moins un atome de silicium.

Les photoamorceurs cationiques sont choisis parmi les borates d'onium (pris à eux seuls ou en mélange entre eux) d'un élément des groupes 15 à 17 de la classification périodique [Chem. & Eng. News, vol.63, N° 5, 26 du 4 février 1985] ou d'un complexe organométallique d'un élément des groupes 4 à 10 de la classification périodique [même référence].

L'entité cationique du borate est sélectionnée parmi :
**(1)** les sels d'onium de formule **(I)** :

   [(R¹)ₙ-A-(R²)ₘ]⁺ (**I**)

   formule dans laquelle :
   - A représente un élément des groupes 15 à 17 tel que par exemple : I, S, Se, P ou N,
   - R¹ représente un radical aryle carbocyclique ou hétérocyclique en C₆-C₂₀, ledit radical hétérocyclique pouvant contenir comme hétéroéléments de l'azote ou du soufre,
   - R² représente R¹ ou un radical alkyle ou alkényle linéaire ou ramifié en C₁-c₃₀; lesdits radicaux R¹ et R² étant éventuellement substitués par un groupement alcoxy en C₁-C₂₅, alkyle en C₁-C₂₅, nitro, chloro, bromo, cyano, carboxy, ester ou mercapto,
   - n est un nombre entier allant de 1 à v + 1, v étant la valence de l'élément A,
   - m est un nombre entier allant de 0 à v - 1 avec n + m = v + 1,
**(2)** les sels d'oxoisothiochromanium décrits dans la demande de brevet WO 90/11303, notamment le sel de sulfonium du 2-éthyl-4-oxoisothiochromanium ou de 2-dodécyl-4-oxoisothio-chromanium,
**(3)** les sels organométalliques de formule **(III)** :

   (L¹L²L³M)^{+q}

   formule dans laquelle :
   - M représente un métal du groupe 4 à 10, notamment du fer, manganèse, chrome, cobalt,
   - L¹ représente 1 ligand lié au métal M par des électrons π, ligand choisi parmi les ligands η³-alkyl, η⁵⁻ cyclopendadiènyl et η⁷- cycloheptratriènyl et les composés η⁶ - aromatiques choisis parmi les ligands η⁶-benzène éventuellement substitués et les composés ayant de 2 à 4 cycles condensés, chaque cycle étant capable de contribuer à la couche de valence du métal M par 3 à 8 électrons π,
   - L² représente un ligand lié au métal M par des électrons π, ligand choisi parmi les ligands η⁷-cycloheptatriènyl et les composés η⁶-aromatiques choisis parmi les ligands η⁶⁻ benzène éventuellement substitués et les composés ayant de 2 à 4 cycles condensés, chaque cycle étant capable de contribuer à la couche de valence du métal M par 6 ou 7 électrons π,
   - L³ représente de 0 à 3 ligands identiques ou différents liés au métal M par des électrons σ, ligand(s) choisi(s) parmi CO et NO₂⁺ ; la charge électronique totale q du complexe à laquelle contribuent L¹, L² et L³ et la charge ionique du métal M étant positive et égale à 1 ou 2.

L'entité anionique borate a pour formule [BXₐ R_{b}]⁻ dans laquelle :
- a et b sont des nombres entiers allant pour a de 0 à 3 et pour b de 1 à 4 avec a + b = 4,
- les symboles X représentent :
   * un atome d'halogène (chlore, fluor) avec a = 0 à 3,
   * une fonction OH avec a = 0 à 2,
- les symboles R sont identiques ou différents et représentent :
   ▷ un radical phényle substitué par au moins un groupement électroattracteur tel que par exemple OCF₃, CF₃, NO₂, CN, et/ou par au moins 2 atomes d'halogène (fluor tout particulièrement), et ce lorsque l'entité cationique est un onium d'un élément des groupes 15 à 17,
   ▷ un radical phényle substitué par au moins un élément ou un groupement électroattracteur notamment atome d'halogène (fluor tout particulièrement), CF₃, OCF₃, NO₂, CN, et ce lorsque l'entité cationique est un complexe organométallique d'un élément des groupes 4 à 10,
   ▷ un radical aryle contenant au moins deux noyaux aromatiques tel que par exemple biphényle, naphtyle, éventuellement substitué par au moins un élément, ou un groupement électroattracteur, notamment un atome d'halogène (fluor tout particulièrement), OCF₃, CF₃, NO₂, CN, quelle, que soit l'entité cationique.

Sans que cela ne soit limitatif, sont données ci-après plus de précisions quant aux sous classes de borate d'onium et de borate de sels organométalliques plus particulièrement préférés dans le cadre de l'utilisation conforme à l'invention.

Selon une première variante préférée de l'invention, les espèces de l'entité anionique borate qui conviennent tout particulièrement sont les suivantes :

| | |
|---|---|
| **1'**: [B(C₆F₅)₄]⁻ | **5'**: [B(C₆H₃(CF₃)₂)₄]⁻ |
| **2'**: [(C₆F₅)₂BF₂]⁻ | **6'**: [B(C₆H₃F₂)₄]⁻ |
| **3'**: [B(C₆H₄CF₃)₄]⁻ | **7'** v [C₆F₅BF₃]⁻ |
| **4'**: [B(C₆F₄OCF₃)₄]⁻. | |

Selon une deuxième variante préférée de l'invention, les sels d'onium (1) utilisables sont décrits dans de nombreux documents notamment dans les brevets US-A-4 026 705, US-A-4 032 673, US-A-4 069 056, US-A-4136 102, US-A-4 173 476. Parmi ceux-ci, on privilégiera tout particulièrement les cations suivants :
[(Φ)₂I]⁺; [C₈H₁₇-O-Φ-I-Φ]⁺; [CH₃-Φ-I-Φ-CH₂CH(CH₃)₂]⁺;
[C₁₂H₂₅-Φ-I-Φ]⁺ ; [(C₈H₁₇-O-Φ)₂I]+; [(C₈H₁₇-O-Φ-I-Φ)]⁺_{;}
[(Φ)₃S]⁺; [(Φ)₂-S-Φ-O-C₈H₁₇]⁺ ; [(CH₃-Φ-I-Φ-CH(CH₃)₂]⁺ ;
[Φ-S-Φ-S-(Φ)₂]⁺ ; [(C₁₂H₂₅-Φ₂ I]⁺; [(CH₃-Φ-I-Φ-OC₂H₅]⁺;
[(C₁₂H₂₅-Φ-I-Φ-CH-(CH₃)₂]⁺.

Selon une troisième variante préférée, les sels organométalliques (4) utilisables sont décrits dans les documents US-A-4 973 722, US-A-4 992 572, EP-A-203 829, EP-A-323 584 et EP-A-354 181. Les sels organométalliques plus volontiers retenus selon l'invention sont notamment :
- le (η⁵ - cyclopentadiènyle) (η⁶ - toluène) Fe⁺,
- le (η⁵ - cyclopentadiènyle) (η⁶ - méthyl-1-naphtalène) Fe⁺,
- le (η⁵ - cyclopentadiènyle) (η⁶ - cumène) Fe⁺,
- le bis (η⁶ - mesitylène) Fe+,
- le bis (η⁶ - benzène) Cr⁺

En accord avec ces trois variantes préférées, on peut citer, à titre d'exemples de photoamorceurs du type borates d'onium, les produits suivants :
[(C₈H₁₇)-O-Φ-I-Φ)]⁺, [B(C₆F₅)₄]⁻;
[C₁₂H₂₅-(D-I-Φ]⁺, [B(C₆F₅)₄]⁻ ;
[(C₈H₁₇-O-Φ)₂I]⁺, [B(C₆F₅)₄]⁻;
[(C₈H₁₇)-O-Φ-I-Φ)]⁺, [B(C₆F₅)₄]-;
[(Φ)₂S-Φ-O-C₈H₁₇]⁺, [B(C₆H₄CF₃)₄]⁻;
[(C₁₂H₂₅-Φ)₂I]⁺, [B(C₆F₅)₄]⁻;
[CH₃-Φ-I-Φ-CH(CH₃)₂]⁺, [B(C₆F₅)₄]⁻ ;
(η⁵ - cyclopentadiènyle) (η⁶ - toluène) Fe⁺, [B(C₆F₅)₄]⁻ ;
(η⁵ - cyclopentadiènyle) (η⁶ - methyl-1-naphtalène) Fe⁺, [B(C₆F₅)₄]⁻ ;
(η⁵ - cyclopentadiènyle) (η⁶ - cumène) Fe⁺, [B(C₆F₅)₄]⁻ ;
[C₁₂H₂₅-Φ)₂I]⁺, [B(C₆H₃(CF₃)₂]⁻ ;
[CH₃-Φ-I-Φ-CH₂CH(CH₃)₂]⁺, [B(C₆F₅)₄]⁻ ;
[CH₃-Φ-I-Φ-CH₂CH(CH₃)₂]⁺, [B(C₆H₃(CF₃)₂)₄]⁻.

Comme autre référence littéraire pour définir les borates d'onium (1) et (2) et les borates de sels organométalliques (4), on peut citer l'ensemble du contenu des demandes de brevet EP 0 562 897 et 0 562 922. Ce contenu est intégralement incorporé par référence dans le présent exposé.

Comme autre exemple de sel d'onium utilisable comme photoamorceur, on peut citer ceux divulgués dans les brevets américains US 4138 255 et US 4 310 469.

On peut également utiliser d'autres photoamorceurs cationiques, e. g. :
- les sels d'iodonium hexafluorophosphate ou hexafluoro-antimonate, tels que :
   - [CH₃-Φ-I-Φ-CH(CH₃)₂]⁺, [PF₆]⁻ ;
   - [CH₃-Φ-I-Φ-CH₂CH(CH₃)₂]⁺, [PF₆]⁻ ;
   - [C₁₂H₂₅-Φ)₂-I]⁺, [PF₆]⁻;
- ou les sels de ferrocénium de ces différents anions.

Le photosensibilisateur contenu au sein de la composition dentaire selon l'invention peut être de nature très variée. Dans le cadre de l'invention celui-ci répond notamment à l'une des formule **(IV)** à **(XXII)** suivantes : 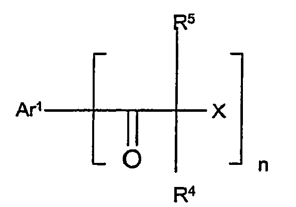
• formule **(IV)**
   dans laquelle :
   - lorsque n = 1, **Ar¹** représente un radical aryle contenant de 6 à 18 atomes de carbone, un radical tétrahydronaphtyle, thiényle, pyridyle ou furyle ou un radical phényle porteur d'un ou plusieurs substituants choisis dans le groupe constitué de F, Cl, Br, CN, OH, les alkyles linéaires ou ramifiés en C₁-C₁₂, - CF³, -OR⁶, -OPhényle, -SR⁶, -SPhényle, -SO₂Phényle, -COOR⁶, -O-(CH₂-CH=CH₂), -O(CH₂H₄-O)ₘ-H, -O(C₃H₆O)ₘ-H, m étant compris entre 1 et 100,
   - lorsque n = 2, **Ar₁** représente un radical arylène en C₆-C₁₂ ou un radical phénylène-T-phénylène, où T représente -O-, -S-, -SO₂- ou -CH₂-, - **X** représente un groupe -OR⁷ ou -OSiR⁸(R⁹)₂ ou forme, avec R⁴, un groupe - O-CH(R¹⁰)-,
   - R₄ représente un radical alkyle linéaire ou ramifié en C₁-C₈ non substitué ou porteur d'un groupe -OH, -OR⁶, acyloxy en C₂-C₈, -CF³, ou -CN, un radical alcényle en C₃ ou C₄, un radical aryle en C₆ à C₁₈_, un radical phénylalkyle en C₇ à C_{9,}
   - **R⁵** a l'une des significations données pour R⁴ ou représente un radical - CH₂CH₂R¹¹, ou encore forme avec R⁴, un radical alkylène en C₂-C₈ ou un radical oxa-alkylène ou aza-alkylène en C₃-C₉,
   - **R⁶** représente un radical alkyle inférieur contenant de 1 à 12 atomes de carbone,
   - **R⁷** représente un atome d'hydrogène, un radical alkyle en C₁-C₁₂, un radical alkyle en C₂-C₆ porteur d'un groupe -OH, -OR⁶ ou CN, un radical alcényle en C₃-C₆, un radical cyclohexyle ou benzyle, un radical phényle éventuellement substitué par un atome de chlore ou un radical alkyle linéaire ou ramifié en C₁-C₁₂, ou un radical tétrahydropyrannyle-2,
   - **R⁸** et **R⁹** sont identiques ou différents et représentent chacun un radical alkyle en C₁-C₄ ou un radical phényle,
   - **R¹⁰** représente un atome d'hydrogène, un radical alkyle en C₁-C₈ ou un radical phényle,
   - **R¹¹** représente un radical -CONH₂, -CONHR⁶, -CON(R⁶)₂, -P(O)(OR⁶)₂ ou pyridyle-2 : 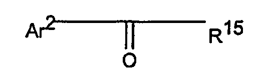
• formule **(V)**
   dans laquelle :
   - Ar² a la même signification que Ar¹ de la formule **(IV)** dans le cas où n =1,
   - **R¹⁵** représente un radical choisi parmi le groupe constitué d'un radical **Ar²,** un radical alkyle linéaire ou ramifié en C₁-C₁₂, un radical cycloalkyle en C₆-C₁₂ et un radical cycloalkyle formant un cycle en C₆-C₁₂ avec le carbone de la cétone ou un carbone du radical **Ar²,** ces radicaux pouvant être substitués par un ou plusieurs substituants choisis dans le groupe constitué de -F, -Cl, -Br, -CN, -OH, -CF₃" -OR⁶, -SR⁶, -COOR⁶, les radicaux alkyles linéaires ou ramifiés en C₁-C₁₂ porteurs éventuellement d'un groupe -OH, -OR⁶ et/ou -CN, et les radicaux alcényles linéaires ou ramifiés en C₁-C₈ ; 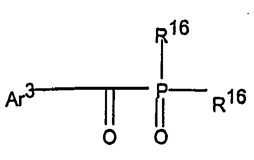
• formule **(VI)**
   dans laquelle :
   - **Ar³** a la même signification que Ar¹ de la formule **(IV)** dans le cas où n = 1,
   - **R¹⁶**, identique ou différent, représente un radical choisi parmi le groupe constitué d'un radical Ar³, un radical -(C=O)-Ar³, un radical alkyle linéaire ou ramifié en C₁-C₁₂, un radical cycloalkyle en C₆-C₁₂, ces radicaux pouvant être substitués par un ou plusieurs substituants choisis dans le groupe constitué de -F, -Cl, -Br, -CN, -OH, -CF₃, -OR⁶, -SR⁶, -COOR⁶, les radicaux alkyles linéaires ou ramifiés en C₁-C₁₂ porteurs éventuellement d'un groupe -OH, -OR⁶ et/ou -CN, et les radicaux alcényles linéaires ou ramifiés en C₁-C₈ ; 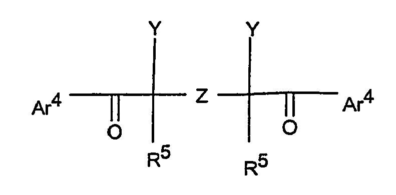
• formule **(VII)**
   dans laquelle :
   - **R⁵,** identiques ou différents, ont les mêmes significations que dans la formule **(III),**
   - **Y,** identiques ou différents, représentent X et/ou R⁴,
   - **Z** représente :
      - une liaison directe,
      - un radical divalent alkylène en C₁-C₆, ou un radical phénylène, diphénylène ou phénylène-T-phénylène (T : alkyle linéaire ou ramifié en C₁-C₁₂), ou encore forme, avec les deux substituants R⁵ et les deux atomes de carbone porteurs de ces substituants, un noyau de cyclopentane ou de cyclohexane, un groupe divalent -O-R¹²-O-, -O-SiR⁸R⁹-O-SiR⁸R⁹-O-, ou -O-SiR⁸R⁹-O-,
   - **R¹²** représente un radical alkylène en C₂-C₈, alcénylène en C₄-C₆ ou xylylène,
   - ou bien encore l'entité : 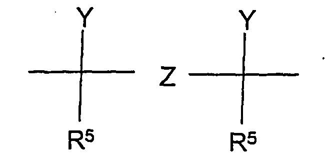 correspond à -O-O-
   - et **Ar⁴** a la même signification que Ar¹ de la formule **(IV)** dans le cas où n = 1.
• famille des thioxanthones de formule **(VIII) :** 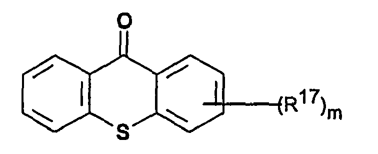
   - m = 0 à 8,
   - **R¹⁷,** identique(s) ou différent(s) substituants sur le(s) noyau(x) aromatique(s), représentent un radical alkyle linéaire ou ramifié en C1-C12, un radical cycloalkyle en C6-C12, un radical Ar¹, un atome d'halogène, un groupement -OH, -CN, -NO₂, -COOR⁶,
      - CHO, O-phényle, -CF₃, -SR⁶, -Sphényle , -SO₂ phényle, O-alcényle ou -SiR⁶₃.
• famille des xanthènes de formule **(IX)** : 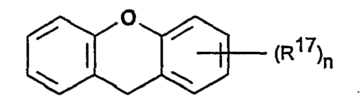 n = 0 à 8
• famille des xanthones de formule **(X):** 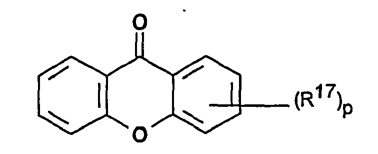 p = 0 à 8
• famille du naphtalène de formule **(XI)**: 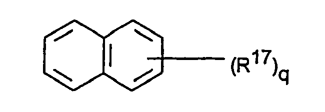
q = 0 à 8
- famille de l'anthracène de formule **(XII) :** 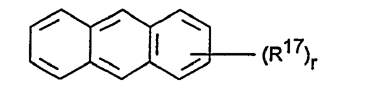
r=0 à 10
- famille du phénanthrène de formule **(XIII):** 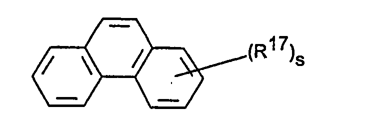
s = 0 à 10
- famille du pyrène de formule **(XIV) :** 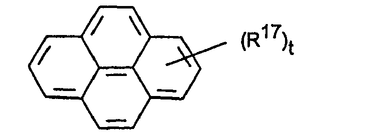
t = 0 à 10
- famille du fluorène de formule **(XV) :** 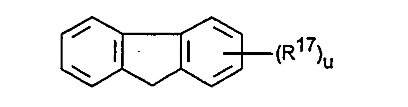
u = 0 à 9
- famille du fluoranthène de formule **(XVI) :** 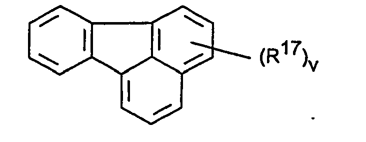
v = 0 à 10
- famille du chrysène de formule **(XVII) :** 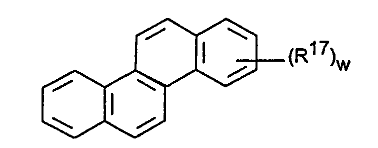
w = 0 à 12
- famille de la fluorène de formule **(XVIII) :** 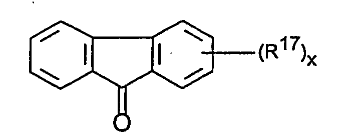
avec x = 0 à 8, par exemple 2,7 dinitro 9-fluorénone,
- famille de la chromone de formule **(XIX) :** 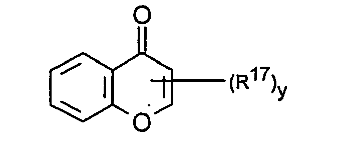
avec y = 0 à 6
- famille de l'éosine de formule **(XX) :** 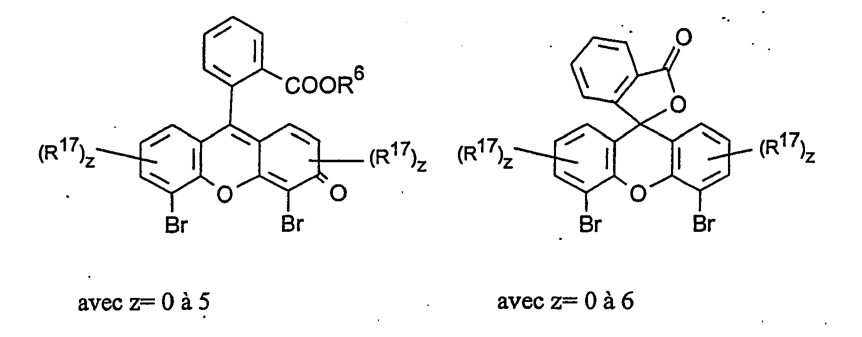
- famille de l'érythrosine de formule **(XXI) :** 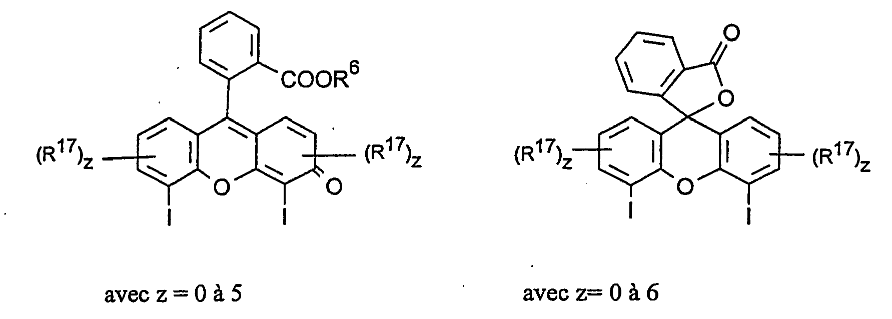
- famille des biscoumarines de formule **(XXII) :** 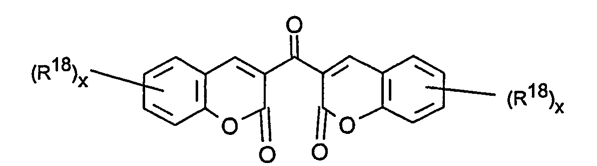
   - **R¹⁸,** identique ou différent, a la même signification que **R¹⁷** ou représente un groupement -NR⁶_{2'} par exemple le 3,3'carbonylbis(7-diéthylaminocoumarin) et le 3,3'-carbonylbis-(7-méthoxy-coumarin).

D'autres sensibilisateurs sont utilisables. Notamment, on peut utiliser les photosensibilisateurs décrits dans les documents US 4,939,069; US 4,278,751; US 4,147,552.

Dans le cadre de la présente invention, les photosensibilisateurs ont une absorption résiduelle de la lumière U.V. comprise entre 200 et 500 nm, de préférence 400 à 500 nm pour les préparations de prothèses dentaires. Pour la restauration dentaire, on préférera un photosensibilisateur ayant une absorption résiduelle de la lumière U.V. au-delà de 400 nm.

Selon une variante préférée, les photosensibilisateurs seront choisis parmi ceux des familles **(IV), (VI), (VII)** et **(VIII).** A titre d'exemples, on citera les phosensibilisateurs suivants:
■ 4,4'diméthoxybenzoïne ;
■ 2-4 diéthyl thioxanthone ;
■ 2-éthylanthraquinone ;
■ 2-méthylanthraquinone ;
■ 1,8-dihydroxyanthraquinone ;
■ dibenzoylperoxyde ;
■ 2,2-diméthoxy-2-phénylacétophénone ; benzoïne ;
■ 2-hydroxy-2méthylpropiophénone ; benzaldéhyde ;
■ 4-(2-hydroxyéthoxy)phényl-(2-hydroxy-2-méthylpropyl) cétone ;
■ benzoylacétone;
■ 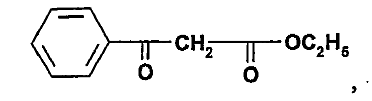
■ 2-isopropylthioxanthone ;
■ 1-chloro-4-propoxythioxanthone ;
■ 4-isopropylthioxanthone ;
■ 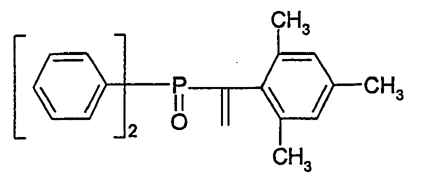
■ 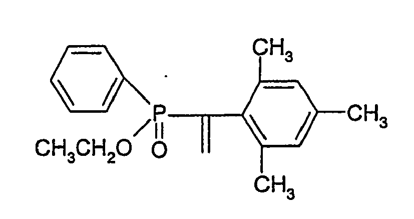
■ et leurs mélanges.

Dans ce cas de traitement de ou des charges siliciées en particulier la silice avec ce type de polymère, le matériau obtenu après réticulation présente une tenue mécanique, un module d'élasticité, et une résistance à la compression nettement améliorés.

Outre les charges de renforcement, des pigments peuvent être utilisés pour teinter la composition dentaire selon l'utilisation envisagée et les groupes ethniques.
Par exemple, on utilise des pigments rouges en présence de microfibres pour les compositions dentaires utilisées pour la préparation de prothèses dentaires afin de simuler les vaisseaux sanguins.
On emploie aussi des pigments à base d'oxydes métalliques (oxydes de fer et/ou titane et/ou aluminium et/ou zirconium, etc.) pour les compositions dentaires utilisées pour la préparation de matériau de restauration, afin d'obtenir un matériau réticulé de couleur ivoire.

D'autres additifs peuvent être incorporés au sein des compositions dentaires selon l'invention. Par exemple, des biocides, des stabilisants, des agents de flaveur, des plastifiants et des promoteurs d'adhérence.
Parmi les additifs envisageables, on utilisera avantageusement des co-réactifs réticulables et/ou polymérisables de type organique. Ces co-réactifs sont liquides à température ambiante ou thermofusibles à température inférieure à 100°C, et chaque co-réactif comprend au moins deux fonctions réactives tels que oxétane-alcoxy, oxétane-hydroxy, oxétane-alcoxysilyle, carboxy-oxétane, oxétane-oxétane, alcénylether-hydroxy, alcénylether-alcoxysilyle, époxy-alcoxy, époxy-alcoxysilyles, dioxolane-dioxolane- alcool, etc.

Les compositions dentaires selon l'invention peuvent être utilisées pour de nombreuses applications dentaires, et en particulier dans le domaine des prothèses dentaires, dans le domaine de la restauration dentaire et dans le domaine des dents provisoires.

La composition dentaire selon l'invention se présente de préférence sous la forme d'un seul produit contenant les différents composants ("*monocomposant*") ce qui facilite sa mise en oeuvre, notamment dans le domaine des prothèses dentaires. Eventuellement, la stabilité de ce produit peut être assurée par des dérivés organiques à fonctions amines selon l'enseignement du document WO 98/07798.

Dans le domaine des prothèses dentaires, le produit sous la forme "monocomposant" peut être déposé à l'aide d'une seringue directement sur le modèle en plâtre ou dans une clé. Puis, il est polymérisé (polymérisation par couches successives possibles) à l'aide d'une lampe UV (spectre lumière visible 200 - 500 nm).
En général, il est possible de réaliser en 10 à 15 min une prothèse dentaire durable et esthétique.

Il est à noter que les produits obtenus à partir de la composition dentaire selon l'invention sont non poreux. Ainsi, après un éventuel polissage à l'aide d'une brosse feutre par exemple, la surface des prothèses dentaires obtenues est lisse et brillante et donc ne nécessite pas d'utilisation de vernis.

Les applications dans le domaine des prothèses dentaires sont essentiellement celles de la prothèse adjointe, que l'on peut diviser en deux types :
- prothèse totale en cas de patient complètement édenté
- prothèse partielle due à l'absence de plusieurs dents se traduisant par soit une prothèse provisoire, soit un appareil squeletté.

Dans le domaine de la restauration dentaire, la composition dentaire selon l'invention peut être utilisée en tant que matériau d'obturation des dents antérieures et postérieures en différentes teintes (par exemple, teintes 'VITA"), rapide et facile à mettre en oeuvre.

La composition dentaire étant non toxique et polymérisable en couches épaisses, il n'est pas indispensable de polymériser le matériau en couches successives. En général, une seule injection de la composition dentaire est suffisante.

- Les préparations pour prothèses dentaires et pour matériaux de restauration sont effectuées selon les techniques usuelles du métier.

Dans le cas d'application de la composition dentaire à une dent, soit la dent peut être prétraitée avec un primaire d'accrochage ou soit la composition dentaire peut être préparée en mélange avec un primaire d'accrochage avant son utilisation.

Les Exemples et Tests suivants sont donnés à titre illustratif. Ils permettent notamment de mieux comprendre l'invention et de faire ressortir certains de ses avantages et d'illustrer quelques unes de ses variantes de réalisation.

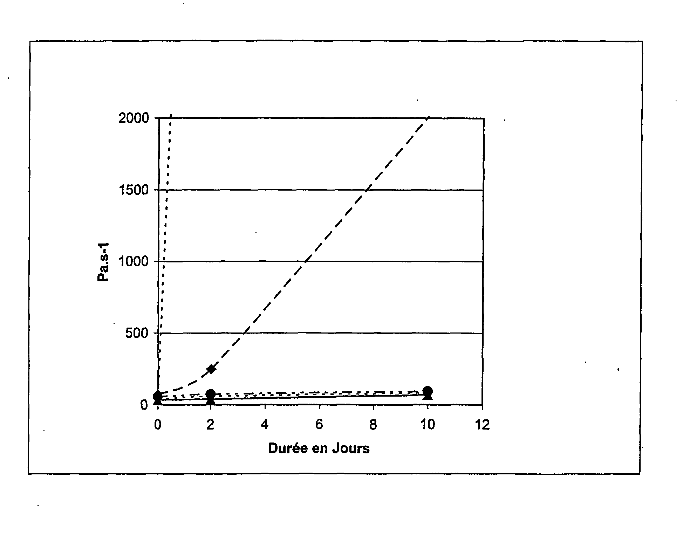

### Description de la figure unique:

La figure unique annexée représente les courbes d'évolution de viscosité en Pa.s⁻¹ en fonction de la durée en jours de stockage.
La légende de cette figure est la suivante :
---◆--- témoin 1
--■--- témoin 2
-▲- formulation 1 selon l'invention (exemple 2.2)
--------- formulation 2 selon l'invention (exemple 2.3)
- -• --- formulation 3 selon l'invention (exemple 2.4)

### EXEMPLES ET TESTS :

Le silicone (1) à fonctionnalité Z époxyde utilisé dans les exemples est (A) :

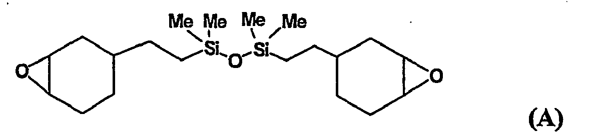

Il est obtenu à partir de 4-vinylcyclohexène-époxyde (VCMX) commercialisé par la société Union Carbide ® (systématiquement distillé avant utilisation) et de tétraméthylhydrogénodisiloxane (M'₂) utilisé est fabriqué par Rhodia Silicones, lui aussi distillé avant utilisation, en présence de catalyseur Pt sur Noir 2S humide est commercialisé par la société ALDRICH sous la référence 33,015-9. Le taux de Platine est de 1,2% en poids. Le taux d'humidité est de 50% en poids.
L'auxiliaire de fonctionnalisation utilisé est l'hydrogénocarbonate de sodium (NaHCO₃).
Le protocole opératoire commun est le suivant :

Dans un réacteur de 100 ml, sont chargés 66 g (531 mmol = 1,05 éq) de VCMX, la quantité de platine nécessaire sous forme du catalyseur Pt sur noir 2S, et éventuellement l'eau et l'hydrogénocarbonate de sodium. Le mélange réactionnel est ensuite chauffé à 90°C. 34 g (506 mmol = 1 éq) de M'₂ sont alors coulés goutte à goutte en 5h dans le réacteur. Lors de la synthèse, l'avancement de la réaction est déterminé par la disparition des motifs ≡SiH et l'éventuelle disparition des fonctions époxy est suivie par dosage potentiométrique. Lorsque tous les motifs ≡SiH ont réagi, le mélange réactionnel est filtré puis dévolatilisé pendant 7 heures sous vide à haute température en présence ou non d'hydrogénocarbonate de sodium. La viscosité, qui est directement corrélée à la perte en fonctions époxydes, est mesurée avant et après dévolatilisation. Le produit obtenu renferme entre 85 et 99 % de silicone (1) de Formule (A) supra et de RNCAS18724.32.-8

Le photoamorceur (3) borate d'onium utilisé pour déclencher la polymérisation des groupes époxy sous UV est décrit dans la demande de brevet EP 0 562 897.

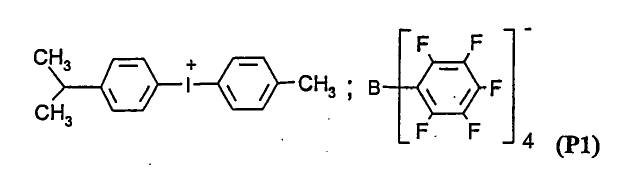

La charge (2) utilisée est un quartz commercialisé par la société Schott sous la référence G018-066 de granulométries 0,7 µm ; 1,5µm ; 3,5µm ;ou 5µm.

Ces quartz sont analysés en Infra rouge par réflexion diffuse et on enregistre trois zones de bandes silanols (silanols libres ; silanols associés 1 et silanols associés 2) entre 3300-4000cm⁻¹. La concentration en sites actifs silanols augmente avec la surface disponible et augmente au fur et à mesure que l'on décroît la taille des particules.

### EXEMPLE 1

### 1.1. Préparation d'une formulation témoin 1 sans dispersant

On charge dans un mélangeur centrifuge de la marque Hauschild^{®}, 25 g de quartz (SiO₂ > 99%) de granulométrie 5 µm commercialisé par Schott, 3 g de trifluorure d'ytterbium, 10 g de résine siloxane ayant une teneur en monomère (A) > 90% obtenue par hydrosilylation du VCMX selon un procédé de fabrication tel que décrit ci-dessus.
On agite 16 s avec le mélangeur centrifuge puis on ajoute 1,25g de système photoamorceur renfermant 30% de photoamorceur P1 et 0,23% de photosensibilisateur à base de chloropropoxythioxanthone CPTX commercialisé par Lambson, le tout en solution dans la résine (A) sans solvant. On agite 16 s avec le mélangeur centrifuge. On rajoute alors 5 g de silice de combustion (SiO₂>99%) puis on agite 16 s. On rajoute 5,75 g grammes de silice de combustion puis on agite 16 s.
La silice de combustion est une silice commercialisée par la société DEGUSSA sous la dénomination OX-50, qui possède une surface spécifique 40 m²/g.
La courbe d'évolution de viscosité est donnée dans la figure unique annexée.
La préparation est gélifiée après 1 mois, stockée à 20°C.

### 1.2. Préparation d'une formulation témoin 2 sans dispersant

On charge dans un mélangeur centrifuge de la marque Hauschild® 25 g de quartz (SiO₂ > 99%) de granulométrie 3 µm commercialisé par Schott, 3 g de trifluorure d'ytterbium, 10 g de résine siloxane ayant une teneur en monomère (A) > 90% obtenue par hydrosilylation du VCMX, tel que décrit supra.
On agite 16 s avec le mélangeur centrifuge puis on ajoute 1,25 g de système photoamorceur dans (A) renfermant 30 % de photoamorceur P1 et 0,23 % de photosensibilisateur (4) à base de chloropropoxy-thioxanthone CPTX commercialisé par Lambson. On agite 16 s avec le mélangeur centrifuge. On rajoute alors 5 g de silice de combustion (SiO₂>99%) puis on agite 16 s. On rajoute 5,75 g de silice de combustion puis on agite 16 s. La silice de combustion est une silice commercialisée par la société DEGUSSA sous la dénomination OX-50 et de surface spécifique égale à 40 m²/g_{.}
La courbe d'évolution de viscosité est donnée dans la figure unique annexée.

La préparation est gélifiée après 48 heures, stockée à 20°C.

### EXEMPLE 2

### 2.1. Préparation selon l'invention

Le dispersant byk^{®}164 (8 g) est mis en solution dans la résine (A) à raison de 4 % et ensuite dévolatilisé de façon à retirer l'acétate de butyle contenu dans le dispersant en chauffant sous vide de 10 mmHg à 60°C pendant 3 heures dans un évaporateur rotatif La concentration de matière active est de 2,4%.

### 2.2. Préparation d'une formulation 1 avec dispersant

On charge dans un mélangeur centrifuge de la marque Hauschild^{®}, 25 g de quartz (SiO₂ > 99%) de granulométrie 5 µm commercialisé par Schott, 3 g de trifluorure d'ytterbium, 1,25 g de solution de dispersant byk^{®} 164 dévolatilisé dans la résine (A) telle que décrite précédemment, on agite 16 s avec le mélangeur centrifuge. On rajoute 9 g de résine (A).
On agite 16 s avec le mélangeur centrifuge, puis on ajoute 1,25 g de système photoamorceur dans (A) renfermant 30% de photoamorceur P1 et 0,23 % de photosensibilisateur CPTX. On agite 16 s avec le mélangeur centrifuge. On rajoute alors 5 g de silice de combustion (SiO₂>99%), puis on agite 16 s. On rajoute 5,5 g de silice de combustion, puis on agite 16 s.
On suit l'évolution de la viscosité au cours du temps.
La préparation ne gélifie pas après 1 mois, stockée à 20°C.
La courbe d'évolution de viscosité est donnée dans la figure unique annexée.

### 2.3. Préparation d'une formulation 2 avec dispersant

On charge dans un mélangeur centrifuge de la marque Hauschild^{®}, 25 g de quartz (SiO₂ > 99%) de granulométrie 3 µm commercialisé par Schott, 3 g de trifluorure d'ytterbium, 1,25 g de solution de dispersant byk^{®} 164 dévolatilisé dans la résine (A), on agite 16 s avec le mélangeur centrifuge. On rajoute 9 g de résine (A).
On agite 16 s avec le mélangeur centrifuge puis on ajoute 1,25 g de système photoamorceur dans (A) renfermant 30 % de photoamorceur P1 et 0,23 % de photosensibilisateur CPTX. On agite 16 s avec le mélangeur centrifuge. On rajoute alors 5 g de silice de combustion (SiO₂>99%), puis on agite 16 s. On rajoute 5,5 g de silice de combustion, puis on agite 16 s.
On suit l'évolution de la viscosité au cours du temps.
La préparation ne gélifie pas après 1 mois, stockée à 20°C.
La courbe d'évolution de viscosité est donnée dans la figure unique annexée.

### 2.4. Préparation d'une formulation 3 avec dispersant

On charge dans un mélangeur centrifuge de la marque Hauschild^{®}, 25 g de quartz (SiO₂ > 99%) de granulométrie 1,5 µm commercialisé par Schott, 3 g de trifluorure d'ytterbium , 1,25 g de solution de dispersant byk^{®} 164 dévolatilisé dans la résine (A), on agite 16 s avec le mélangeur centrifuge. On rajoute 9 g de résine (A).
On agite 16 s avec le mélangeur centrifuge, puis on ajoute 1,25 g de système photoamorceur dans (A) renfermant 30% de photoamorceur P1 et 0,23 % de photosensibilisateur à base de chloropropoxythioxanthone CPTX. On agite 16 s avec le mélangeur centrifuge.
On rajoute alors 5 g de silice de combustion (SiO₂>99%), puis on agite 16 s.
On rajoute 5,5g grammes de silice de combustion, puis on agite 16 s.
On suit l'évolution de la viscosité au cours du temps.
La préparation ne gélifie pas après 1 mois, stockée à 20°C.
La courbe d'évolution de viscosité est donnée dans la figure unique annexée.

La viscosité est mesurée en plaçant la pâte de composite dans l'entre fer plan/plan d'un rhéomètre et avec un gradient de cisaillement de 4s⁻¹. La composition prend un caractère Newtonien avec l'ajout de dispersant. La viscosité n'évolue pas même sous fort gradient de cisaillement.

### EXEMPLE 3

### Photopolymérisation des formulations 1, 2 et 3 (§ 2.2, 2.3, 2.4 supra de restauration dentaire

Les formulations réticulent sur une épaisseur de 3mm en 40s d'irradiation avec une lampe Optilux^{®} Demetron. La dureté Vickers mesurée après photoréticulation est de 50 pour chacune des trois formulations. Le module de flexion et la résistance à la flexion mesurée selon la norme ISO4049 sont respectivement de 5 GPa et de 80 MPa.

### EXEMPLE 4

### 4.1. Préparation d'une formulation avec dispersant phosphate (830 ppm): contre-exemple 4

On charge dans un mélangeur tripale sous agitation lente (< 10 tours/minute), 125 g de monomère (A) et 15 g de solution de Solsperse^{®} 36000 commercialisé par la société AVECM à 4 % dans le monomère A. On mélange pendant 5 mn. On rajoute 18 g de système photoamorceur renfermant 30 % de photoamorceur P1 et 0,23 % de photosensibilisateur à base de chloropropoxythioxanthone CPTX. On mélange pendant 5 mn à température ambiante.
On ajoute le quartz (SiO₂ > 99 %) de granulométrie 3,5 µm commercialisé par Schott, soit 378,5 g et 45 g de trifluorure d'ytterbium. On mélange pendant environ une heure à température ambiante jusqu'à obtenir un mélange visqueux fluide homogène. On rajoute 137 g de silice de combustion OX50 petit à petit pendant 2 heures par ajouts successifs de 15 g. On conserve le mélange en cartouches de 5 g après dépotage dans une salle climatisée à 25°C.

### 4.2. Préparation d'une formulation avec dispersant sans phosphate (830 ppm) exemple 4

On répète la même opération que précédemment en substituant les 15 g de solution de Solsperse^{®} 360000 par 15 g de Disperbyk^{®} 164 0 4 %, avec un indice d'amine de 20 mg de KOH/g de dispersant.
On observe ensuite au bout de combien de mois les produits stockés en cartouche gélifient.
Le produit stocké avec dispersant phosphate gélifie en 3 mois à 25°C. Au bout d'un an, le produit avec dispersant avec indice d'amine ≤ 100 mg de potasse /g de dispersant, est toujours utilisable et non gélifié. Il réticule en moins de 1 mn sur 3 mm avec un module de flexion voisin de 10 GPa à la flexion de 80 MPa.

## Revendications

1. Composition dentaire comprenant :
**(1)** au moins un composé réactif par voie cationique et sous activation de préférence actinique;
**(2)** au moins une charge dentaire présente dans une proportion d'au moins 10% en poids par rapport au poids total de la composition ;
**(3)** au moins un dispersant présent à raison de 50 ppm à 1 %, dont l'indice d'amine est inférieur ou égal à 100 mg de potasse par gramme de dispersant et sélectionné dans le groupe comprenant les copolymères polyuréthanne/acrylate ; éventuellement salifiés par un alkylammonium;
**(4)** au moins un photoamorceur cationique ; et
**(5)** et éventuellement au moins un photosensibilisateur.

2. Composition selon la revendication 1 **caractérisée en ce que** la charge dentaire (2) est présente à raison de 10 à 85 % en poids.

3. Composition dentaire selon l'une quelconque des revendications 1 à 2, **caractérisée en ce que** le dispersant (3) est présent à raison de 100 à 5000 ppm.

4. Composition dentaire selon l'une quelconque des revendications 1 à 3, **caractérisée en ce que** le composé réactif par voie cationique (1) est choisi dans le groupe de monomères et/ou (co)polymèreres comprenant :
les époxy, les vinyles éthers, les oxétanes, les spiroorthocarbonates, les spiro-orthoesters et leurs associations.

5. Composition dentaire selon l'une quelconque des revendications 1 à 4, **caractérisée en ce que** le composé réactif par voie cationique (1) est constitué par au moins un oligomère ou polymère silicone réticulable et/ou polymérisable, liquide à température ambiante ou thermofusible à température inférieure à 100 °C, et comprenant:
□ au moins un motif de formule (FS) : 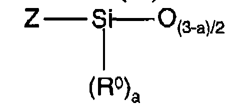 dans laquelle :
- a = 0, 1 ou 2,
- R⁰, identique ou différent, représente un radical alkyle, cycloalkyle, aryle, vinyle, hydrogéno, alcoxy, de préférence un alkyle inférieur en C₁-C_{6,}
- Z, identique ou différent, est un substituant organique comportant au moins une fonction réactive époxy, et/ou alcénylether et/ou oxétane et/ou dioxolane et/ou carbonate,
□ et au moins deux atomes de silicium.

6. Composition dentaire selon la revendication 5, **caractérisée en ce que** Z est un substituant organique Z1 comportant au moins une fonction réactive époxy, et/ou dioxolane, et de préférence au moins une fonction réactive époxy.

7. Composition selon la revendication 5, **caractérisée en ce que** l'oligomère ou polymère **(1)** comporte en outre d'autres fonctions réactives Z telles que les fonctions réactives Z2 alcénylether, oxétane et/ou carbonate.

8. Composition selon la revendication 6 ou 7, **caractérisée en ce que** la ou les fonctions réactives de Z1 sont choisies parmi les radicaux suivants :

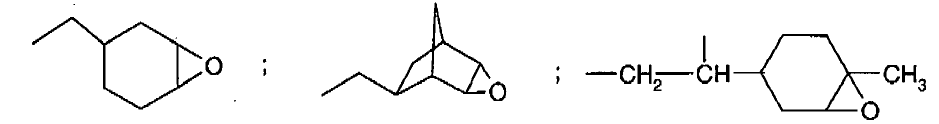

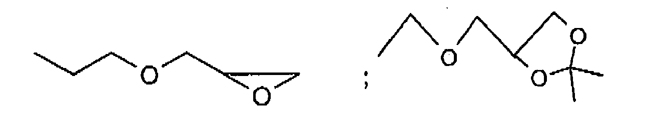

9. Composition dentaire selon l'une quelconque des revendications 1 à 10, **caractérisée en ce que** le photoamorceur est de type borate et est choisi parmi ceux de formule :
Δ dont l'entité cationique du borate est sélectionnée parmi :
**(1)** les sels d'onium de formule **(I) :**
[(R¹)ₙ - A - (R²)ₘ]⁺ **(I)**
formule dans laquelle :
• A représente un élément des groupes 15 à 17 tel que par exemple : I, S, Se, P ou N,
• R¹ représente un radical aryle carbocyclique ou hétérocyclique en C₆-C₂₀, ledit radical hétérocyclique pouvant contenir comme hétéroéléments de l'azote ou du soufre,
• R² représente R¹ ou un radical alkyle ou alkényle linéaire ou ramifié en C₁-C₃₀ ; lesdits radicaux R¹ et R² étant éventuellement substitués par un groupement alcoxy en C₁-C₂₅, alkyle en C₁-C₂₅, nitro, chloro, bromo, cyano, carboxy, ester ou mercapto,
• n est un nombre entier allant de 1 à v + 1, v étant la valence de l'élément A,
• m est un nombre entier allant de 0 à v - 1 avec n + m = v + 1,
(2) les sels d'oxoisothiochromanium ;
(3) les sels organométalliques de formule **(III)** :
(L¹L²L³M)^{+q}
formule dans laquelle :
• M représente un métal du groupe 4 à 10, notamment du fer, manganèse, chrome, cobalt,
• L¹ représente 1 ligand lié au métal M par des électrons π, ligand choisi parmi les ligands η³-alkyl, η⁵⁻ cyclopendadiènyl et η⁷⁻ cycloheptratriènyl et les composés η⁶ - aromatiques choisis parmi les ligands η⁶-benzène éventuellement substitués et les composés ayant de 2 à 4 cycles condensés, chaque cycle étant capable de contribuer à la couche de valence du métal M par 3 à 8 électrons π,
• L² représente un ligand lié au métal M par des électrons π, ligand choisi parmi les ligands η⁷-cycloheptatriènyl et les composés η⁶-aromatiques choisis parmi les ligands η⁶- benzène éventuellement substitués et les composés ayant de 2 à 4 cycles condensés, chaque cycle étant capable de contribuer à la couche de valence du métal M par 6 ou 7 électrons π,
• L³ représente de 0 à 3 ligands identiques ou différents liés au métal M par des électrons σ, ligand(s) choisi(s) parmi CO et NO₂⁺ ; la charge électronique totale q du complexe à laquelle contribuent L¹, L² et L³ et la charge ionique du métal M étant positive et égale à 1 ou 2 ;
Δ dont l'entité anionique borate a pour formule [BXₐ R_{b}]⁻ dans laquelle :
- a et b sont des nombres entiers allant pour a de 0 à 3 et pour b de 1 à 4 avec a + b = 4,
- les symboles X représentent :
* un atome d'halogène (chlore, fluor) avec a = 0 à 3,
* une fonction OH avec a = 0 à 2,
- les symboles R sont identiques ou différents et représentent :
▷ un radical phényle substitué par au moins un groupement électroattracteur tel que par exemple OCF₃, CF₃, NO₂, CN, et/ou par au moins 2 atomes d'halogène (fluor tout particulièrement), et ce lorsque l'entité cationique est un onium d'un élément des groupes 15 à 17,
▷ un radical phényle substitué par au moins un élément ou un groupement électroattracteur notamment atome d'halogène (fluor tout particulièrement), CF₃, OCF₃, NO₂, CN, et ce lorsque l'entité cationique est un complexe organométallique d'un élément des groupes 4 à 10,
▷ un radical aryle contenant au moins deux noyaux aromatiques tel que par exemple biphényle, naphtyle, éventuellement substitué par au moins un élément ou un groupement électroattracteur, notamment un atome d'halogène dont le fluor en particulier, OCF₃, CF₃, NO₂, CN, quelle que soit l'entité cationique.

10. Composition selon la revendication 9, **caractérisée en ce que** le photoamorceur est choisi parmi le groupe constitué par :
[(C₈H₁₇)-O-Φ-I-Φ)]⁺, [B(C₆F₅)₄]⁻;
[C₁₂H₂₅-Φ-I-Φ]⁺, [B(C₆F₅)₄)⁻ ;
[(C₈H₁₇-O-Φ)₂I]⁺, [B(C₆F₅)₄]⁻ ;
[(C₈H₁₇)-O-Φ-I-Φ)]⁺, [B(C₆F₅)₄]⁻;
[(Φ)₂S-Φ-O-C₈H₁₇]⁺, [B(C₆H₄CF₃)₄]⁻;
[(C₁₂H₂₅-Φ)₂I]⁺, [B(C₆F₅)₄]⁻;
[CH₃-Φ-I-Φ-CH(CH₃)₂]⁺, [B(C₆F₅)₄]⁻;
(η⁵ - cyclopentadiènyle) (η⁶ - toluène) Fe⁺, [B(C₆F₅)₄]⁻ ;
(η⁵ - cyclopentadiènyle) (η⁶ - methyl-1-naphtalène) Fe⁺, [B(C₆F₅)₄]⁻;
(η⁵ - cyclopentadiènyle) (η⁶ - cumène) Fe⁺, [B(C₆F₅)₄]⁻ ;
[C₁₂H₂₅-Φ)₂I]⁺, [B(C₆H₃(CF₃)₂]⁻ ;
[CH₃-Φ-I-Φ-CH₂CH(CH₃)₂]⁺, [B(C₆F₅)₄]⁻;
[CH₃-Φ-I-Φ-CH₂CH(CH₃)₂]⁺, [B(C₆H₃(CF₃)₂)₄]⁻.

11. Composition dentaire selon l'une quelconque des revendications 1 à 10, **caractérisée en ce qu'**elle comporte un photosensibilisateur **(5)** hydrocarboné aromatique à un ou plusieurs noyaux aromatiques substitués ou non, ayant une absorption résiduelle de la lumière comprise entre 200 et 500 nm.

12. Composition selon la revendication 11, **caractérisée en ce que** le photosensibilisateur est choisi parmi le groupe constitué de :
■ 4,4'diméthoxybenzoïne ;
■ 2-4 diéthyl thioxanthone ;
■ 2-éthylanthraquinone ;
■ 2-méthylanthraquinone ;
■ 1,8-dihydroxyanthraquinone ;
■ dibenzoylperoxyde ;
■ 2,2-diméthoxy-2-phénylacétophénone ; benzoïne ;
■ 2-hydroxy-2méthylpropiophénone ; benzaldéhyde ;
■ 4-(2-hydroxyéthoxy)phényl-(2-hydroxy-2-méthylpropyl) cétone ;
■ benzoylacétone;
■ 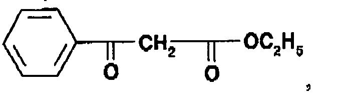
■ 2-isopropylthioxanthone,
■ 1-chloro-4-propoxythioxanthone
■ 4-isopropylthioxanthone,
■ 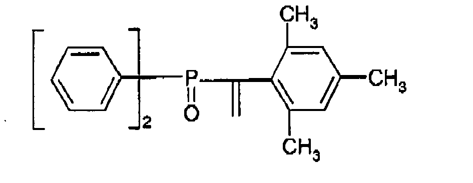 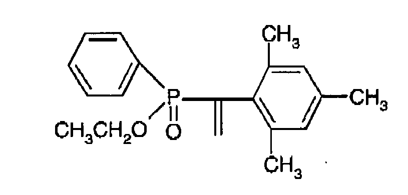
■ et leurs mélanges.

13. Composition dentaire selon l'une quelconque des revendications 1 à 12, **caractérisée en ce que** l'oligomère et/ou polymère silicone (1) est constituée par au moins une silicone de formule moyenne suivante :

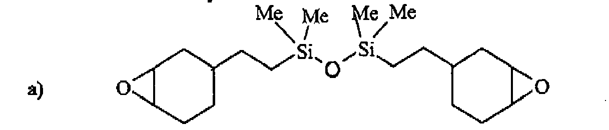

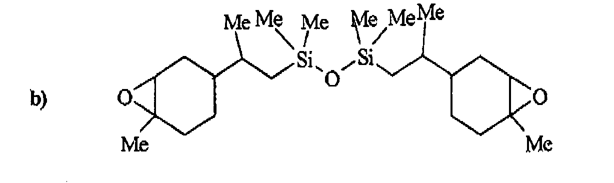

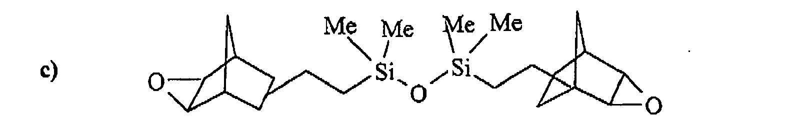

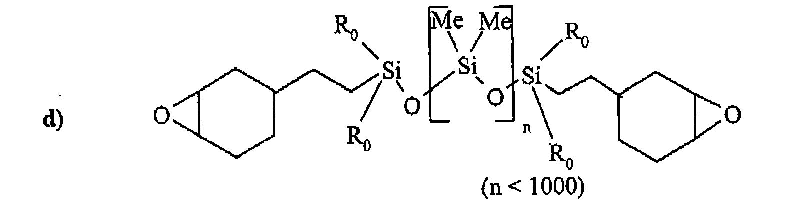

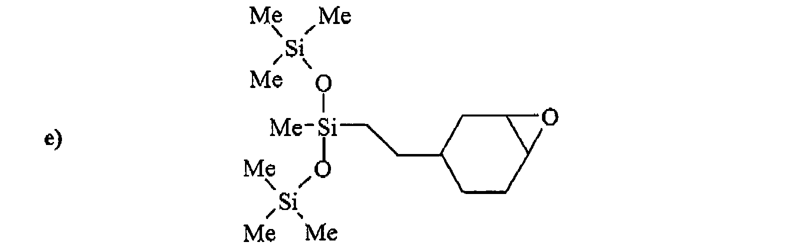

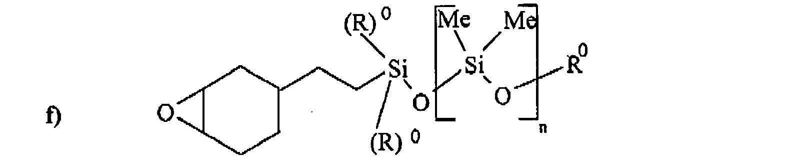

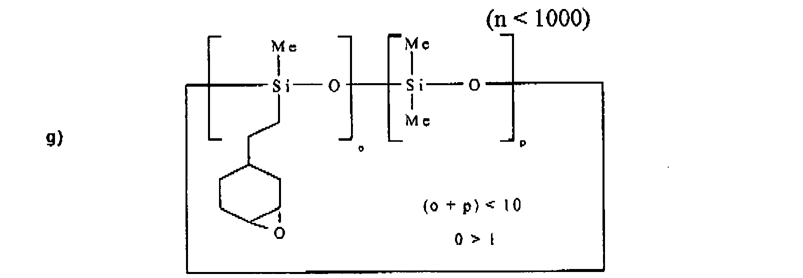

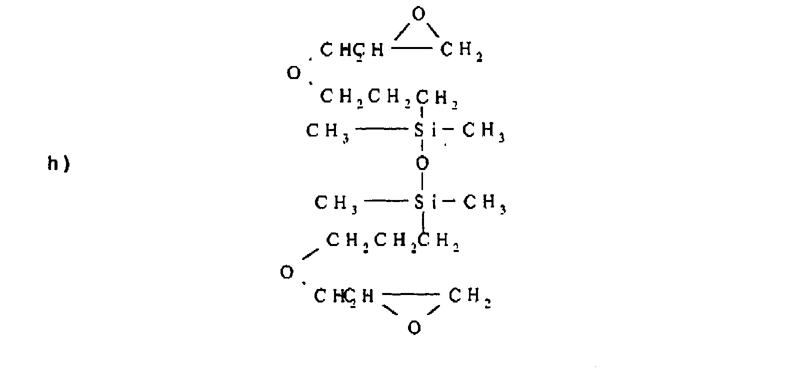

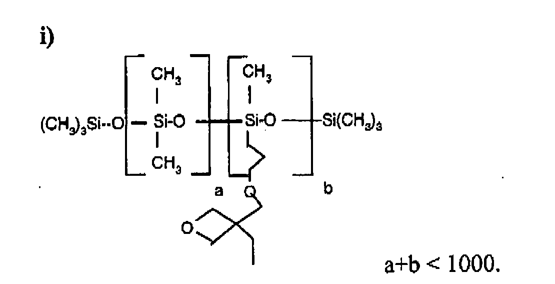

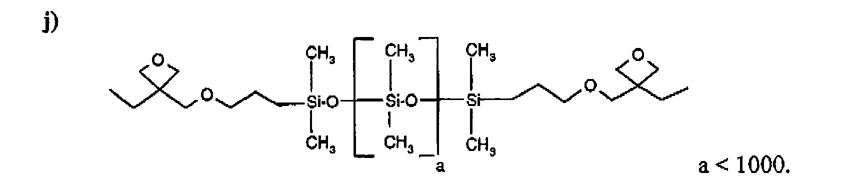

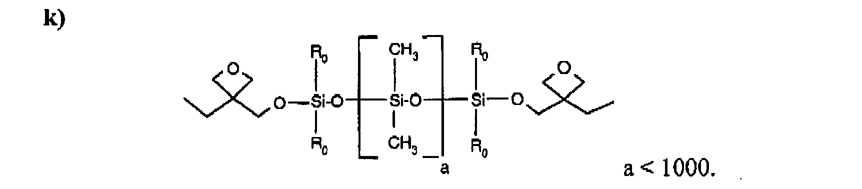

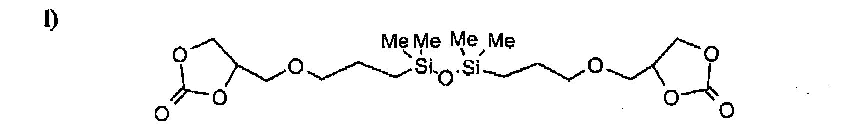

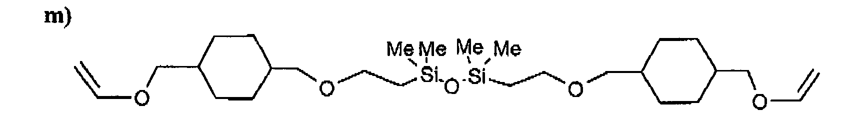

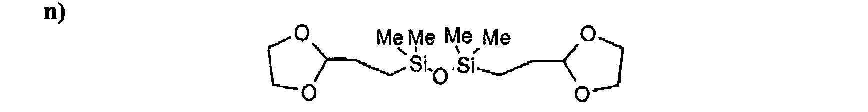

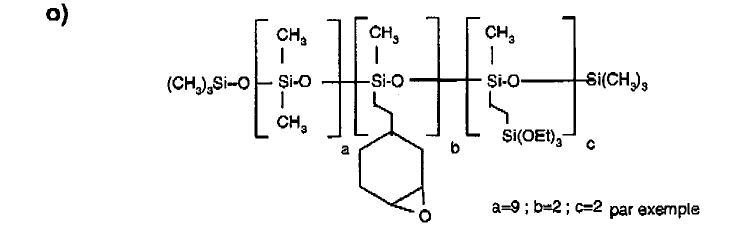

14. Utilisation d'une composition dentaire selon l'une quelconque des revendications 1 à 13, pour la réalisation de prothèses dentaires.

15. Utilisation d'une composition dentaire selon l'une quelconque des revendications 1 à 13, pour la restauration dentaire.

16. Prothèse dentaire susceptible d'être obtenue à partir d'une composition selon l'une quelconque des revendications 1 à 13.

17. Matériau de restauration dentaire susceptible d'être obtenu à partir d'une composition selon l'une quelconque des revendications 1 à 13.

## Claims

1. Dental composition comprising:
(1) at least one cationically reactive compound under preferably actinic activation;
(2) at least one dental filler present in a proportion of at least 10% by weight relative to the total weight of the composition;
(3) at least one dispersant present in a proportion of from 50 ppm to 1%, in which the amine number is less than or equal to 100 mg of potassium hydroxide per gram of dispersant and selected from the group comprising polyurethane/acrylate copolymers; optionally salified with an alkylammonium;
(4) at least one cationic photoinitiator; and
(5) optionally at least one photosensitizer.

2. Composition according to Claim 1, **characterized in that** the dental filler (2) is present in a proportion of from 10 to 85% by weight.

3. Dental composition according to any one of Claims 1 to 2, **characterized in that** the dispersant (3) is present in a proportion of from 100 to 5000 ppm.

4. Dental composition according to any one of Claims 1 to 3, **characterized in that** the cationically reactive compound (1) is chosen from the group of monomers and/or (co)polymers comprising:
epoxys, vinyl ethers, oxetanes, spiroorthocarbonates and spiroorthoesters, and combinations thereof.

5. Dental composition according to any one of Claims 1 to 4, **characterized in that** the cationically reactive compound (1) consists of at least one crosslinkable and/or polymerizable silicone oligomer or silicone polymer, which is liquid at room temperature or thermofusible at a temperature below 100°C, and comprising:
□ at least one unit having the formula (FS): 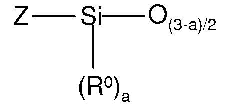 in which:
- a = 0, 1 or 2,
- R⁰, which may be identical or different, represents an alkyl, cycloalkyl, aryl, vinyl, hydrogeno or alkoxy radical, preferably a C₁-C₆ lower alkyl,
- Z, which may be identical or different, is an organic substituent comprising at least one epoxy and/or alkenyl ether and/or oxetane and/or dioxolane and/or carbonate function,
□ and at least two silicon atoms.

6. Dental composition according to Claim 5, **characterized in that** Z is an organic substituent Z1 comprising at least one epoxy and/or dioxolane reactive function, and preferably at least one epoxy reactive function.

7. Composition according to Claim 5, **characterized in that** the oligomer or polymer (1) also comprises other reactive functions Z such as alkenyl ether, oxetane and/or carbonate reactive functions Z2.

8. Composition according to Claim 6 or 7, **characterized in that** the reactive function or functions of Z1 are chosen from the following radicals:

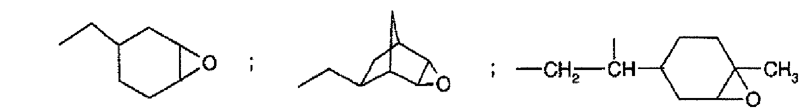

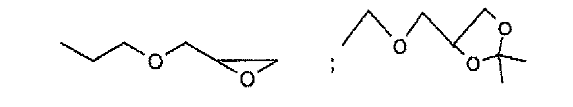

9. Dental composition according to any one of Claims 1 to 10, **characterized in that** the photoinitiator is of borate type and is chosen from those of the formula:
△ in which the cationic species of the borate is selected from:
**(1)** the onium salts of formula **(I):**
[(R¹)ₙ - A - (R²)ₘ](**I**)
in which formula:
• A represents an element from groups 15 to 17, for instance: I, S, Se, P or N,
• R¹ represents a C₆-C₂₀ carbocyclic or heterocyclic aryl radical, said heterocyclic radical possibly containing nitrogen or sulphur as hetero elements,
• R² represents R¹ or a linear or branched C₁-C₃₀ alkyl or alkenyl radical; said radicals R¹ and R² being optionally substituted with a C₁-C₂₅ alkoxy, C₁-C₂₅ alkyl, nitro, chloro, bromo, cyano, carboxyl, ester or mercapto group,
• n is an integer ranging from 1 to v + 1, v being the valency of the element A,
• m is an integer ranging from 0 to v - 1 with n + m = v + 1,
**(2)** oxoisothiochromanium salts;
(3) organometallic salts of formula (III):
(L¹L²L³M)^{+q}
in which formula:
• M represents a metal from groups 4 to 10, especially iron, manganese, chromium or cobalt,
• L¹ represents a ligand linked to the metal M via π electrons, the ligand being chosen from the ligands η³-alkyl, η⁵-cyclopentadienyl and η⁷-cycloheptatrienyl and the η⁶-aromatic compounds chosen from the optionally substituted η⁶-benzene ligands and the compounds containing from 2 to 4 fused rings, each ring being capable of contributing to the valency layer of the metal M via 3 to 8 π electrons,
• L² represents a ligand linked to the metal M via π electrons, the ligand being chosen from the η⁷-cycloheptatrienyl ligands and the η⁶-aromatic compounds chosen from the optionally substituted η⁶-benzene ligands and the compounds containing from 2 to 4 fused rings, each ring being capable of contributing to the valency layer of the metal M via 6 or 7 π electrons,
• L³ represents from 0 to 3 identical or different ligands linked to the metal M via σ electrons, this or these ligand(s) being chosen from CO and NO₂⁺; the total electronic charge q of the complex toward which L¹, L² and L³ contribute and the ionic charge of the metal M being positive and equal to 1 or 2;
Δ in which the anionic borate species has the formula [BXₐR_{b}]⁻ in which:
- a and b are integers ranging for a from 0 to 3 and for b from 1 to 4 with a + b = 4,
- the symbols X represent:
* a halogen atom (chlorine or fluorine) with a = 0 to 3,
* an OH function with a = 0 to 2,
- the symbols R are identical or different and represent:
▷ a phenyl radical substituted with at least one electron-withdrawing group, for instance OCF₃, CF₃, NO₂ or CN, and/or with at least 2 halogen atoms (most particularly fluorine), this being the case when the cationic species is an onium of an element from groups 15 to 17,
▷ a phenyl radical substituted with at least one element or an electron-withdrawing group, especially a halogen atom (most particularly fluorine), CF₃, OCF₃, NO₂ or CN, this being the case when the cationic species is an organometallic complex of an element from groups 4 to 10,
▷ an aryl radical containing at least two aromatic nuclei, for instance biphenyl or naphthyl, optionally substituted with at least one element or an electron-withdrawing group, especially a halogen atom including fluorine, in particular, OCF₃, CF₃, NO₂ or CN, irrespective of the cationic species.

10. Composition according to Claim 9, **characterized in that** the photoinitiator is chosen from the group consisting of:
[(C₈H₁₇)-O-Φ-I-Φ)]⁺, [B(C₆F₅)₄]⁻;
[C₁₂H₂₅-Φ-I-Φ]⁺, [B(C₆F₅)₄]⁻;
[(C₈H₁₇-O-Φ)₂I]⁺, [B(C₆F₅)₄]⁻;
[(C₈H₁₇)-O-Φ-I-Φ)]⁺, [B(C₆F₅)₄]⁻;
[(Φ)₂S-Φ-O-C₈H₁₇]⁺, [B(C₆H₄CF₃)₄]⁻;
[(C₁₂H₂₅-Φ)₂I]⁺, [B(C₆F₅)₄]⁻;
[CH₃-Φ-I-Φ-CH(CH₃)₂]⁺, [B(C₆F₅)₄]⁻;
(η⁵-cyclopentadienyl) (η⁶-toluene)Fe⁺, [B(C₆F₅)₄]⁻;
(η⁵-cyclopentadienyl) (η⁶-methyl-1-naphthalene)Fe⁺, [B(C₆F₅)₄]⁻;
(η⁵-cyclopentadienyl) (η⁶-cumene)Fe⁺, [B(C₆F₅)₄]⁻;
[(C₁₂H₂₅-Φ)₂I]⁺, [B(C₆H₃(CF₃)₂]⁻;
[CH₃-Φ-I-Φ-CH₂CH(CH₃)₂]⁺, [B(C₆F₅)₄]⁻;
[CH₃-Φ-I-Φ-CH₂CH(CH₃)₂]⁺, [B(C₆H₃(CF₃)₂)₄]⁻.

11. Dental composition according to any one of Claims 1 to 10, **characterized in that** it comprises an aromatic hydrocarbon-based photosensitizer (5) having one or more substituted or unsubstituted aromatic nuclei and having a residual light absorption of between 200 and 500 nm.

12. Composition according to Claim 11, **characterized in that** the photosensitizer is chosen from the group formed by:
• 4,4'-dimethoxybenzoin;
• 2,4-diethylthioxanthone;
• 2-ethylanthraquinone;
• 2-methylanthraquinone;
• 1,8-dihydroxyanthraquinone;
• dibenzoyl peroxide;
• 2,2-dimethoxy-2-phenylacetophenone; benzoin;
• 2-hydroxy-2-methylpropiophenone; benzaldehyde;
• 4-(2-hydroxyethoxy)phenyl-(2-hydroxy-2-methylpropyl) ketone;
• benzylacetone;
• 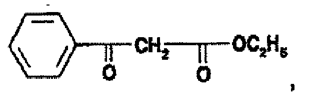
• 2-isopropylthioxanthone;
• 1-chloro-4-propoxythioxanthone;
• 4-isopropylthioxanthone;
• 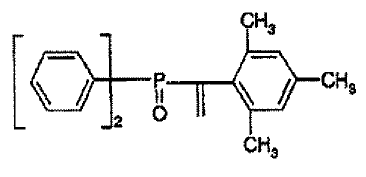
• 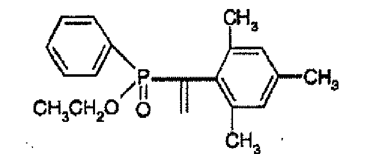
• and mixtures thereof.

13. Dental composition according to any one of Claims 1 to 12, **characterized in that** the silicone oligomer and/or silicone polymer (1) consists of at least one silicone of the following average formulae:

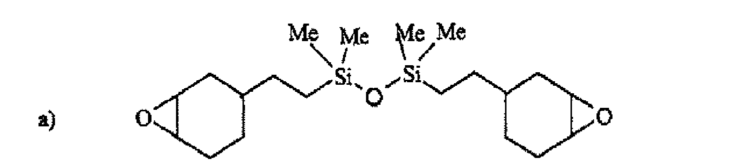

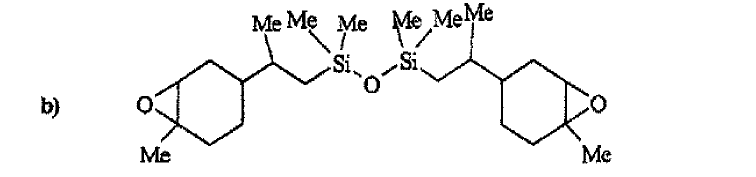

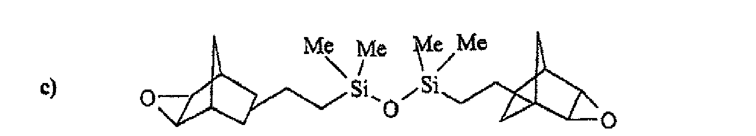

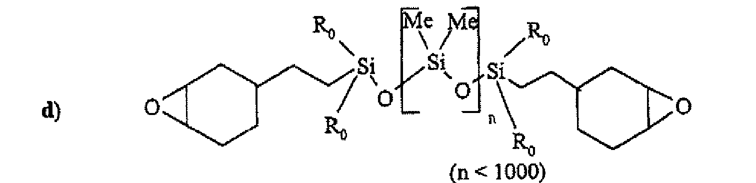

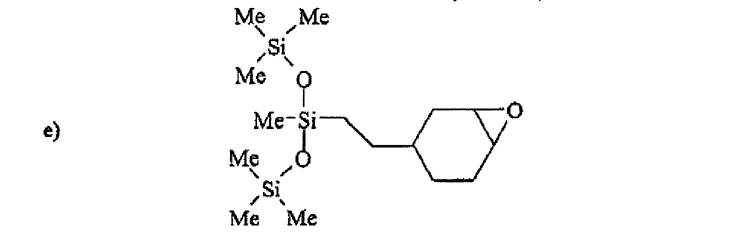

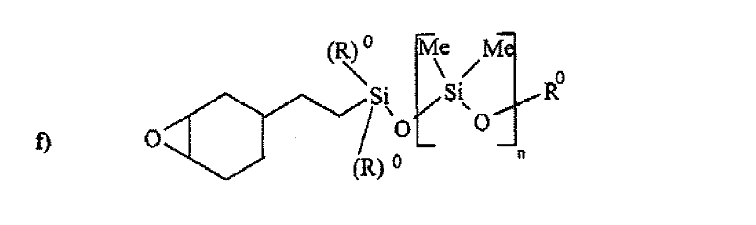

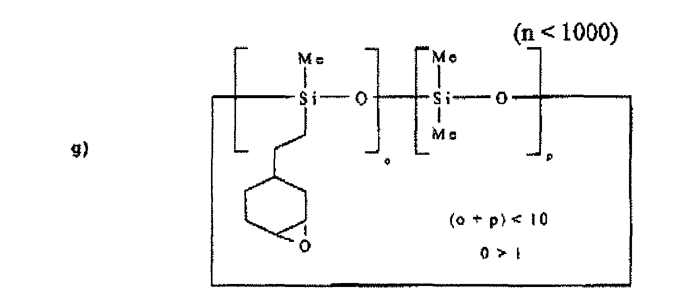

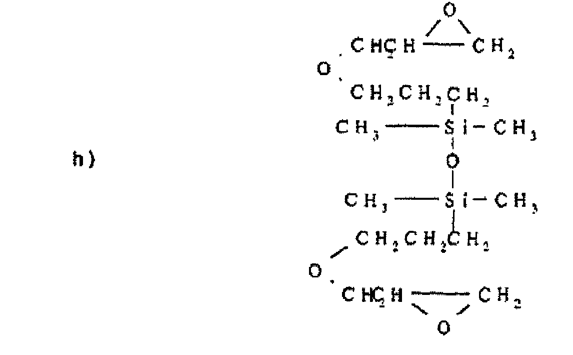

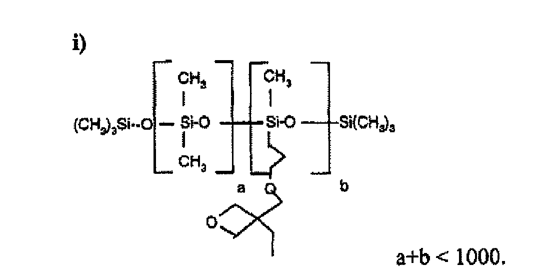

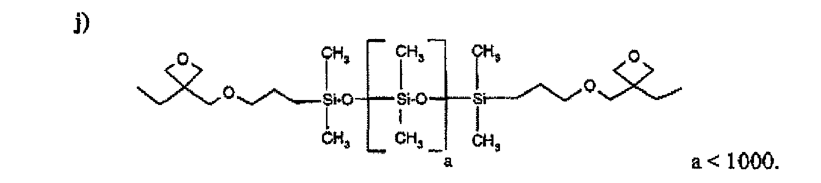

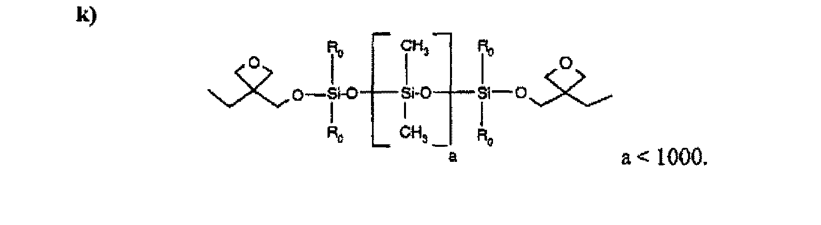

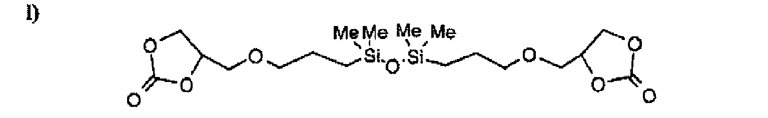

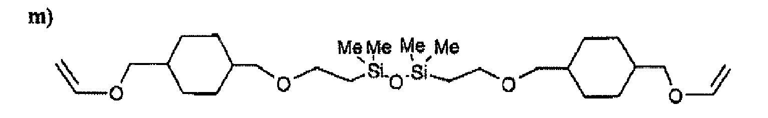

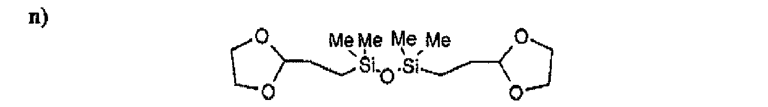

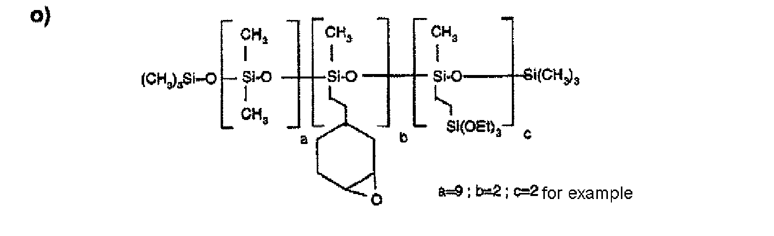

14. Use of a dental composition according to any one of Claims 1 to 13, for the manufacture of dental prostheses.

15. Use of a dental composition according to any one of Claims 1 to 13, for dental restoration.

16. Dental prosthesis that may be obtained from a composition according to any one of Claims 1 to 13.

17. Dental restoration material that may be obtained from a composition according to any one of Claims 1 to 13.

## Patentansprüche

1. Dentalzusammensetzung, umfassend:
(1) mindestens eine Verbindung, die auf kationischem Wege und unter vorzugsweise durch Strahlung hervorgerufener Aktivierung reaktiv ist;
(2) mindestens einen Dentalfüllstoff, der bezogen auf das Gesamtgewicht der Zusammensetzung in einem Anteil von mindestens 10 Gew.-% vorhanden ist;
(3) mindestens ein Dispersionsmittel, das mit 50 ppm bis zu 1 % vorhanden ist und dessen Aminindex kleiner oder gleich 100 mg Kaliumhydroxid pro Gramm Dispersionsmittel ist und aus der Gruppe ausgewählt ist, welche die Polyurethan-Acrylat-Copolymere umfasst, die gegebenenfalls mit einem Alkylammonium ein Salz bilden;
(4) mindestens einen kationischen Photoinitiator; und
(5) gegebenenfalls mindestens einen Photosensibilisator.

2. Zusammensetzung nach Anspruch 1, **dadurch gekennzeichnet, dass** der Dentalfüllstoff (2) mit 10 bis 85 Gew.-% vorhanden ist.

3. Dentalzusammensetzung nach einem der Ansprüche 1 bis 2, **dadurch gekennzeichnet, dass** das Dispersionsmittel (3) mit 100 bis 5000 ppm vorhanden ist.

4. Dentalzusammensetzung nach einem der Ansprüche 1 bis 3, **dadurch gekennzeichnet, dass** die auf kationischem Wege reaktive Verbindung (1) aus der Gruppe der Monomere und/oder (Co)polymere ausgewählt wird, die Folgende umfasst:
die Epoxy, die Vinylether, die Oxetane, die Spiroorthocarbonate, die Spiroorthoester und ihre Assoziate.

5. Dentalzusammensetzung nach einem der Ansprüche 1 bis 4, **dadurch gekennzeichnet, dass** die auf kationischem Wege reaktive Verbindung (1) mindestens aus einem vernetzbaren und/oder polymerisierbaren Silikonoligomer oder -polymer besteht, das bei Umgebungstemperatur flüssig oder bei einer Temperatur unterhalb von 100 °C wärmeschmelzbar ist und folgendes umfasst:
□ mindestens eine Einheit der Formel (FS): 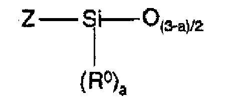 in der:
- a = 0, 1 oder 2,
- R⁰, identisch oder verschieden, für einen Alkyl-, Cycloalkyl-, Aryl-, Vinyl-, Wasserstoff- oder Alkoxyrest steht, vorzugsweise für ein niederes Alkyl mit C₁-C₆;
- Z, identisch oder verschieden, ein organischer Substituent ist, der mindestens eine reaktive Epoxy- und/oder Alkenylether- und/oder Oxetan- und/oder Dioxolan- und/oder Carbonatfunktion umfasst,
□ und mindestens zwei Siliciumatome.

6. Dentalzusammensetzung nach Anspruch 5, **dadurch gekennzeichnet, dass** Z ein organischer Substituent Z1 ist, der mindestens eine reaktive Epoxy- und/oder Dioxolanfunktion und vorzugsweise mindestens eine reaktive Epoxyfunktion umfasst.

7. Zusammensetzung nach Anspruch 5, **dadurch gekennzeichnet, dass** das Oligomer oder Polymer (1) des Weiteren andere reaktive Funktionen Z umfasst, wie z. B.
die reaktiven Alkenylether-, Oxetan- und/oder Carbonatfunktionen Z2.

8. Zusammensetzung nach Anspruch 6 oder 7, **dadurch gekennzeichnet, dass** die reaktive(n) Funktion(en) Z1 aus den folgenden Resten ausgewählt ist/sind:

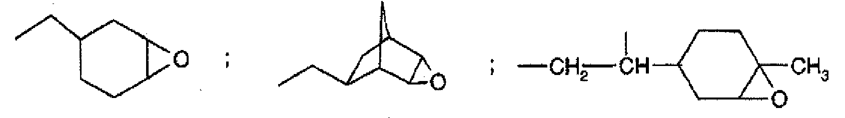

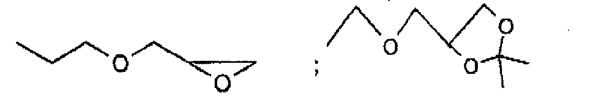

9. Dentalzusammensetzung nach einem der Ansprüche 1 bis 10, **dadurch gekennzeichnet, dass** der Photoinitiator vom Borattyp ist und aus denen der folgenden Formel ausgewählt ist:
Δ deren kationische Borateinheit ausgewählt ist aus:
(1) den Oniumsalzen der Formel (I):
[(R¹)ₙ-A-(R²)ₘ]⁺ (I)
wobei in der Formel:
• A für ein Element der Gruppen 15 bis 17 steht, wie zum Beispiel: I, S, Se, P oder N,
• R¹ für einen carbocyclischen oder heterocyclischen C₆-C₂₀-Arylrest steht, wobei dieser heterocyclische Rest als Heteroelemente Stickstoff oder Schwefel enthalten kann,
• R² für R¹ oder einen linearen oder verzweigten C₁-C₃₀-Alkyl- oder Alkenylrest steht, wobei die Reste R¹ und R² gegebenenfalls durch einen C₁-C₂₅-Alkoxyrest, einen C₁-C₂₅-Alkylrest, Nitro-, Chlor-, Brom-, Cyano-, Carboxy-, Ester- oder Mercarptorest substituiert sein können,
• n eine ganze Zahl im Bereich von 1 bis v+1 ist, wobei v die Valenz des Elements A ist,
• m eine ganze Zahl im Bereich von 0 bis v-1 ist, mit n+m = v+1
(2) den Oxoisothiochromaniumsalzen;
(3) den Organometallsalzen der Formel (III):
(L¹L²L³M)^{+q}
wobei in der Formel:
• M für ein Metall der Gruppe 4 bis 10 steht, insbesondere für Eisen, Mangan, Chrom oder Cobalt,
• L¹ für 1 Liganden steht, der über π-Elektronen an das Metall M gebunden ist, wobei der Ligand aus den η³-Alkyl-, η⁵-Cyclopentadienyl- und η⁷-Cycloheptatrienylliganden und den aromatischen η⁶-Verbindungen ausgewählt ist, die ihrerseits aus den gegebenenfalls substituierten η⁶-Benzolliganden und den Verbindungen mit 2 bis 4 kondensierten Ringen ausgewählt sind, wobei jeder Ring 3 bis 8 π-Elektronen zur Valenzschicht des Metalls M beitragen kann,
• L² für einen Liganden steht, der über π-Elektronen an das Metall M gebunden ist, wobei der Ligand aus den η⁷-Cycloheptatrienylliganden und den aromatischen η⁶-Verbindungen ausgewählt ist, die ihrerseits aus den gegebenenfalls substituierten η⁶-Benzolliganden und den Verbindungen mit 2 bis 4 kondensierten Ringen ausgewählt sind, wobei jeder Ring 6 oder 7 π-Elektronen zur Valenzschicht des Metalls M beitragen kann,
• L³ für 0 bis 3 identische oder verschiedene Liganden steht, die über σ-Elektronen mit dem Metall M verbunden sind, wobei der/die Ligand (en) aus CO und NO₂⁺ ausgewählt ist/sind; wobei die elektrische Gesamtladung q des Komplexes, an dem L¹, L² und L³ beteiligt sind, sowie die ionische Ladung des Metalls M positiv und gleich 1 oder 2 ist;
Δ deren anionische Borateinheit durch die Formel [BXₐR_{b}]⁻wiedergegeben ist, in der:
- a und b ganze Zahlen im Bereich von 0 bis 3 für a und von 1 bis 4 für b sind, mit a+b = 4,
- die Symbole X für Folgendes stehen:
* ein Halogenatom (Chlor, Fluor) mit a = 0 bis 3,
* eine OH-Funktion mit a = 0 bis 2,
- die Symbole R identisch oder verschieden sind und für Folgendes stehen:
► einen Phenylrest, der durch mindestens eine elektronenziehende Gruppe wie zum Beispiel OCF₃, CF₃, NO₂, CN und/oder mindestens 2 Halogenatome (insbesondere Fluor) substituiert ist, und das, wenn die kationische Einheit ein Onium eines Elements der Gruppen 15 bis 17 ist,
► einen Phenylrest, der durch mindestens ein elektronenziehendes Element oder eine elektronenziehende Gruppe, insbesondere durch ein Halogenatom (insbesondere Fluor), CF₃, OCF₃, NO₂, CN, substituiert ist, und das, wenn die kationische Einheit ein organometallischer Komplex eines Elements der Gruppen 4 bis 10 ist,
► einen Arylrest, der mindestens zwei aromatische Kerne enthält, wie zum Beispiel Biphenyl, Naphthyl, die gegebenenfalls, unabhängig von der kationischen Einheit, durch mindestens ein elektronenziehendes Element oder eine elektronenziehende Gruppe, insbesondere durch ein Halogenatom, und darunter insbesondere Fluor, OCF₃, CF₃, NO₂, CN substituiert sind.

10. Zusammensetzung nach Anspruch 9, **dadurch gekennzeichnet, dass** der Photoinitiator aus der Gruppe ausgewählt ist, die aus den Folgenden besteht:
[(C₈H₁₇)-O-Φ-I-Φ)]⁺, [B(C₆F₅)₄]⁻;
[C₁₂H₂₅-Φ-I-Φ]⁺, [B(C₆F₅)₄]⁻;
[(C₈H₁₇-O-Φ)₂I]⁺, [B(C₆F₅)₄]⁻;
[(C₈H₁₇)-O-Φ-I-Φ)]⁺, [B(C₆F₅)₄]⁻;
[(Φ)₂S-Φ-O-C₈H₁₇]⁺, [B(C₆H₄CF₃)₄]⁻;
[(C₁₂H₂₅-Φ)₂I]⁺, [B(C₆F₅)₄]⁻;
[CH₃-Φ-I-Φ-CH (CH₃)₂]⁺, [B(C₆F₅)₄]⁻;
(η⁵-Cyclopentadienyl) (η⁶-Toluol)Fe⁺, [B(C₆F₅)₄]⁻;
(η⁵-Cyclopentadienyl) (η⁶-Methyl-1-naphthalen)Fe⁺, [B(C₆F₅)4]⁻;
(η⁵-Cyclopentadienyl) (η⁶-Cumol)Fe⁺, B(C₆F₅)₄]⁻;
[C₁₂H₂₅-Φ)₂I]⁺, [B(C₆H₃(CF₃)₂]⁻;
[CH₃-Φ-I-Φ-CH₂CH (CH₃)₂]⁺, [B(C₆F₅)₄]⁻;
[CH₃-Φ-I-Φ-CH₂CH(CH₃)₂]⁺, [B(C₆H₃(CF₃)₂)₄]⁻.

11. Dentalzusammensetzung nach einem der Ansprüche 1 bis 10, **dadurch gekennzeichnet, dass** sie einen Photosensibilisator (5) aus einem aromatischen Kohlenwasserstoff mit einem oder mehreren substituierten oder unsubstituierten aromatischen Kernen mit einer Restabsorption von Licht zwischen 200 und 500 nm umfasst.

12. Zusammensetzung nach Anspruch 11, **dadurch gekennzeichnet, dass** der Photosensibilisator aus der Gruppe ausgewählt ist, die aus den Folgenden besteht:
■ 4,4'-Dimethoxybenzoin;
■ 2-4-Diethylthioxanthon;
■ 2-Ethylanthrachinon;
■ 2-Methylanthrachinon;
■ 1,8-Dihydroxyanthrachinon;
■ Dibenzoylperoxid;
■ 2,2-Dimethoxy-2-phenylacetophenon; Benzoin;
■ 2-Hydroxy-2-methylpropiophenon; Benzaldehyd;
■ 4-(2-Hydroxyethoxy)phenyl-(2-hydroxy-2-methylpropyl)keton;
■ Benzoylaceton;
■ 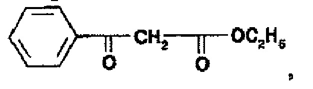
■ 2-Isopropylthioxanthon,
■ 1-Chlor-4-propoxythioxanthon
■ 4-Isopropylthioxanthon, 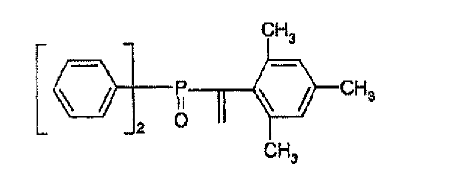 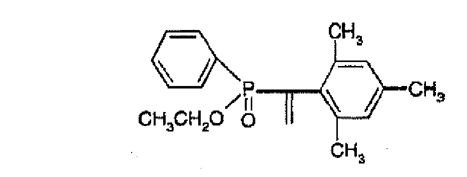
■ und ihren Mischungen.

13. Dentalzusammensetzung nach einem der Ansprüche 1 bis 12, **dadurch gekennzeichnet, dass** das Silikonoligomer oder -polymer (1) aus mindestens einem Silikon mit einer der folgenden mittleren Formel besteht:

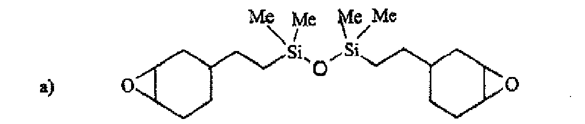

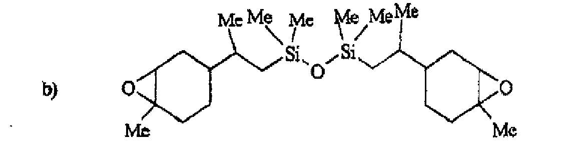

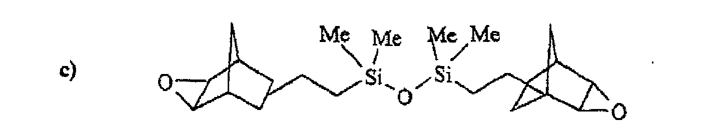

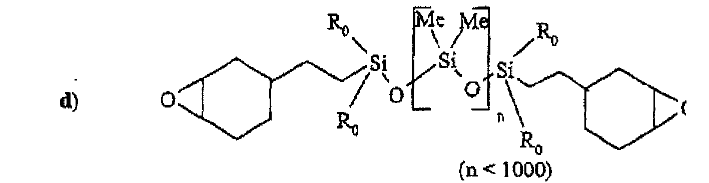

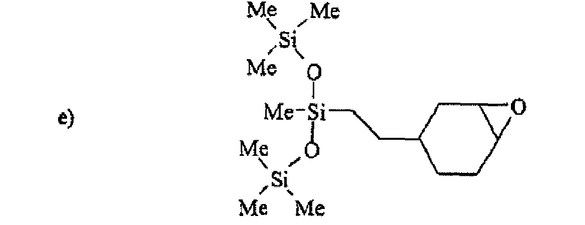

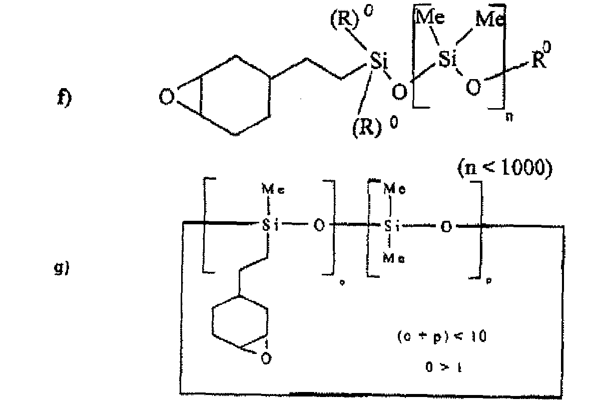

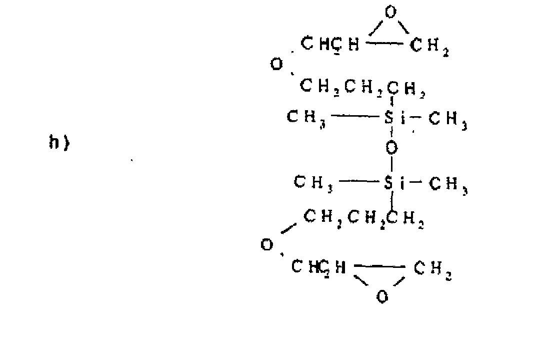

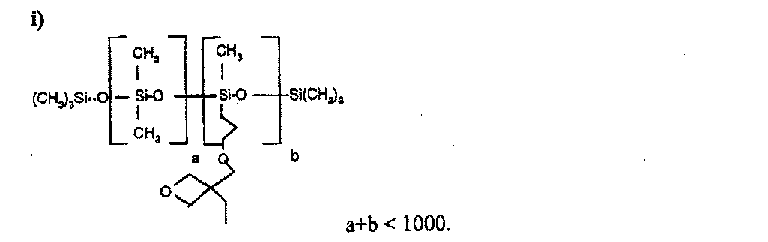

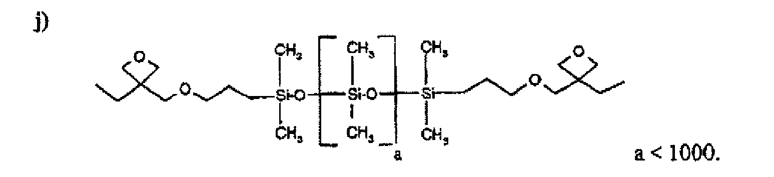

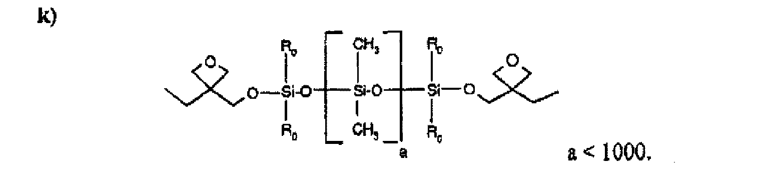

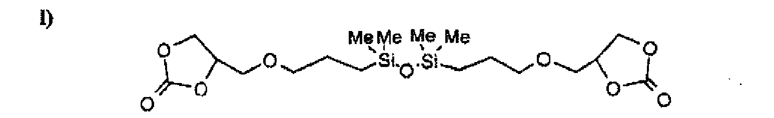

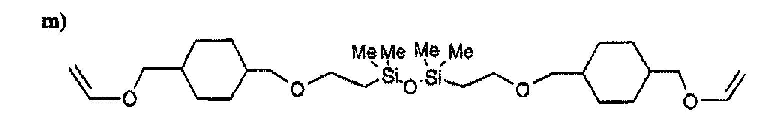

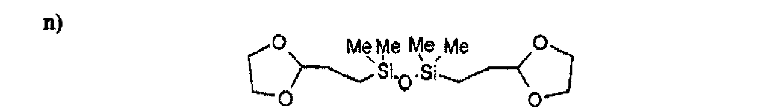

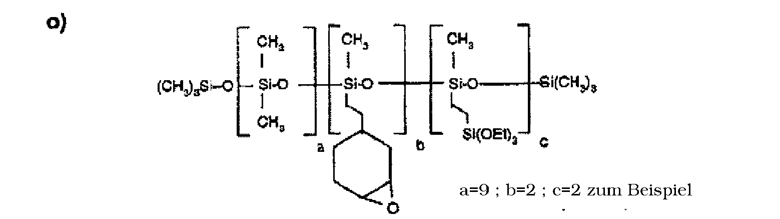

14. Verwendung einer Dentalzusammensetzung nach einem der Ansprüche 1 bis 13 für die Ausführung von Zahnprothesen.

15. Verwendung einer Dentalzusammensetzung nach einem der Ansprüche 1 bis 13, für die Zahnwiederherstellung.

16. Zahnprothese, die ausgehend von einer Zusammensetzung nach einem der Ansprüche 1 bis 13 erhalten werden kann.

17. Material für die Zahnwiederherstellung, das ausgehend von einer Zusammensetzung nach einem der Ansprüche 1 bis 13 erhalten werden kann.
